# EUROPEAN PATENT SPECIFICATION

(11) **EP 1 919 398 B1**
(45) Date of publication and mention of the grant of the patent: **05.03.2014**
(21) Application number: 06800359.9
(22) Date of filing: 26.07.2006
(51) Int. Cl.: A61F 2/24

(54) **ELLIPTICAL IMPLANTABLE DEVICE**
ELLIPTISCHE IMPLANTIERBARE VORRICHTUNG
DISPOSITIF ELLIPTIQUE IMPLANTABLE

(30) Priority: 29.07.2005 US 703772 P
(43) Date of publication of application: 14.05.2008
(73) Proprietor: Cook Medical Technologies LLC, Bloomington, IN 47404 (US)
(72) Inventor: AGNEW, Charles, W., West Lafayette, IN 47906 (US)
(74) Representative: Jehan, Robert
(86) International application number: PCT/US2006/029045
(87) International publication number: WO 2007/016165

(56) References cited:
- WO-A-2004/080352
- WO-A-2005/011534
- WO-A2-2004/019814
- US-A1- 2005 137 681

## Description

### RELATED APLICATIONS

This application claims foreign priority to U.S. Provisional Patent Application No. 60/703,772, entitled "Elliptical Implantable Device," filed July 29, 2005.

### TECHNICAL FIELD

The present invention relates to medical devices. More particularly, the invention relates to medical devices for implantation in a body site.

### BACKGROUND

Many vessels in animals transport fluids from one body location to another. Frequently, fluid flows in a substantially unidirectional manner along the length of the vessel. For example, veins in the body transport blood to the heart and arteries carry blood away from the heart. Various implantable medical devices can be implanted by minimally invasive methods to deliver these medical devices within the lumen of a body vessel. These devices are advantageously inserted intravascularly, for example from an implantation catheter. Implantable medical devices can function as a replacement valve, or restore native valve function by bringing incompetent valve leaflets into closer proximity. Such devices may include an expandable frame configured for implantation in the lumen of a body vessel, such as the heart, an artery or a vein. Valve devices may further comprise features that provide a valve function, such as opposable leaflets.

Dynamic fluctuations in the shape of the vessel lumen, such as a vein, pose challenges to the design of implantable prosthetic devices that conform to the interior shape of the lumen. In the venous system, the flow velocity and diameter of veins does not remain constant at a given systemic vascular resistance. Instead, the shape of vein lumens can fluctuate dynamically in response to the respiration, muscle movement, body position, central venous pressure, arterial inflow and calf muscle pump action of a mammalian subject. Muscles surrounding veins can impart an elliptical cross sectional shape to a vein lumen. The veins also provide a volume capacitance organ. For example, an increase of almost 100% in the diameter of the common femoral vein has been observed in human patients simply by rotation of the patient by about 40 degrees, corresponding to a four-fold increase in blood flow volume. Moneta et al., "Duplex ultrasound assessment of venous diameters, peak velocities and flow patterns," J. Vasc. Surg. 1988; 8; 286-291. Therefore, the shape of a lumen of a vein, which is substantially elliptical in cross-section, can undergo dramatic dynamic change as a result of varying blood flow velocities and volumes therethrough, presenting challenges for designing implantable intraluminal prosthetic devices that more closely conform to the changing shape of the vein lumen. The heart and arteries under go similar static and dynamic distortion to the shape of the heart and arteries, respectively, due to changes in blood flow velocity and volume and the like.

Implantable devices for treating diseases in dynamic vessels, such as veins, are often not designed to conform to the elliptical shape of the vessel or to be responsive to dynamic changes in the shape of the vessel at the implantation site. For example, implantable prosthetic stents or valves often have a circular cross section with the same resistance to radial compression in any radial direction. Similarly, implantable device configurations can be unresponsive to dynamic changes of the vessel cross-section, and can locally distort the shape of the body vessel.

There exists a need in the art for an implantable prosthetic device that is capable of better conforming to the shape of the vessel lumen having an elliptical shape, and being more responsive to dynamic changes in body vessel lumen shape. Such a device can closely simulate the normal vessel shape and responsiveness, as well as normal valve function, while being capable of implantation with excellent biocompatibility.

Elliptical valves are known from WO 2004/019814 and US 2005/0137681.

### SUMMARY

Implantable prosthetic valves having an elliptical cross-section are provided herein. Preferably, a prosthetic valve is shaped and configured to substantially conform to the shape of a vein. The prosthetic valve can have any suitable configuration. Preferably, a prosthetic valve comprises an elliptical support means to provide an elliptical shape to the outer surface of the prosthetic valve and a means for regulating fluid flow through the prosthetic valve.

The elliptical support means can comprise any structural feature that imparts an elliptical cross section to the outer surface of the prosthetic valve. Examples of the elliptical support means can include the cross-linking or stiffening of a tubular tissue construct, a molded plastic support structure, and a metallic frame comprising a plurality of struts and bends. Preferably, the elliptical support means also provides a desired degree of rigidity or flexibility to an elliptical prosthetic valve. The elliptical support means can be formed from any biocompatible material, including a polymer, tissue, metal or a combination thereof. Preferably, the elliptical support means is a support structure formed from a molded thermoformable polymer, although other materials can be used. An elliptical support means can also define an interior lumen shape forming a conduit for fluid flow through the lumen. Preferably, the lumen extends along a longitudinal axis of the elliptical support and connects to a valve orifice.

The prosthetic valve can further comprise a means for regulating fluid within a body vessel. Desirably, the means for regulating fluid is a flexible structure adapted to regulate fluid flow through the prosthetic valve by moving in response to fluid flow within a body vessel, such as a flexible tubular fluid conduit or one or more valve leaflets defining a valve orifice. The means for regulating fluid is preferably one or more moveable valve leaflets. For example, the valve can comprise one or more leaflets attached to an elliptical support and configured to allow fluid flow in substantially antegrade direction through the lumen. The valve leaflets are preferably formed from a suitably flexible material that is moveable in response to fluid flow within a body vessel. A valve orifice is preferably defined by the coaptation of flexible edges of two or more opposable leaflets attached to the elliptical support. The valve orifice can have an open position permitting fluid to flow through the valve in a first direction and a closed position substantially preventing fluid flow past the valve in the opposite direction. Preferably, the valve orifice is moveable between the open position and the closed position as one or more valve leaflets move in response to changes in the fluid direction within the body vessel. Retrograde fluid flow can be diverted by the closed valve orifice into adjacent valve pocket regions formed between each valve leaflet and the wall of the body vessel.

In the invention, a compressible prosthetic valve device is provided having varying resistance to radial compression depending on the direction of the compression. For example, the prosthetic valve may be adapted to collapse or compress along a symmetry plane containing the longitudinal axis of a body vessel, for example by folding out of a flat plane perpendicular to the body vessel. The prosthetic valve comprises an elliptical support structure or support frame with one or more collapse points. Collapse points can be positioned to desirably improve the flow dynamics of a valve. For example, collapse points can be positioned and configured to promote the emptying of retrograde fluid from valve pocket regions when a valve orifice is opened. Incorporation of collapse points in the elliptical support can increase the flexibility of the prosthetic valve in one or more radial directions. For example, positioning pairs of collapse points in an elliptical support can increase the flexibility of the frame along a first radial direction without substantially changing the flexibility in a second radial direction. Increased flexibility of an elliptical support is desirable, for example, to change the shape of the elliptical support in response to changes in fluid flow or body vessel constriction or expansion. Collapse points may be formed by any suitable method that provides a desired increase in the flexibility of a portion of the elliptical support or a support frame, such as providing a reduced-thickness region, or providing a hinge. The collapse points are preferably paired on opposite sides of an interior lumen defined by the elliptical support or support frame. Collapse points can be aligned with one of a first radial axis or the second radial axis of a valve orifice formed in the elliptical support.

A method of making a prosthetic valve device for implantation in a body vessel is described. The method includes providing an elliptical support means having an elliptical cross-sectional shape and defining an interior humen therethrough and providing a flexible member. The method further includes connecting a means for regulating fluid flow to the elliptical support means. In one aspect, the elliptical support means can also be a means for regulating fluid flow. For instance, a flexible tubular member having an elliptical cross section is one example of an elliptical support means. The flexible tubular member can have a tapered end for regulating fluid flow. Alternatively, a means for regulating fluid flow can be attached to an elliptical support structure so that the flexible member is operable to regulate fluid flow through the opening.

Advantages of the present invention will become more apparent to those skilled in the art from the following description of the preferred embodiments of the invention which have been shown and described by way of illustration. As will be realized, the invention is capable of other and different embodiments, and its details are capable of modification in various respects. Accordingly, the drawings and description are to be regarded as illustrative in nature and not as restrictive.

### BRIEF DESCRIPTION OF THE DRAWINGS

**FIG. 1A** is a perspective view of an elliptical valve device embodiment in a vessel in an open configuration; **FIG. 1B** is a top view of the elliptical valve device embodiment shown in **FIG. 1A****;**
**FIG. 2A** is a perspective view of an elliptical valve device embodiment in a vessel in **FIG. 1A** in the closed configuration; **FIG. 2B** is a top view of the elliptical valve device embodiment shown in **FIG. 2A****;**

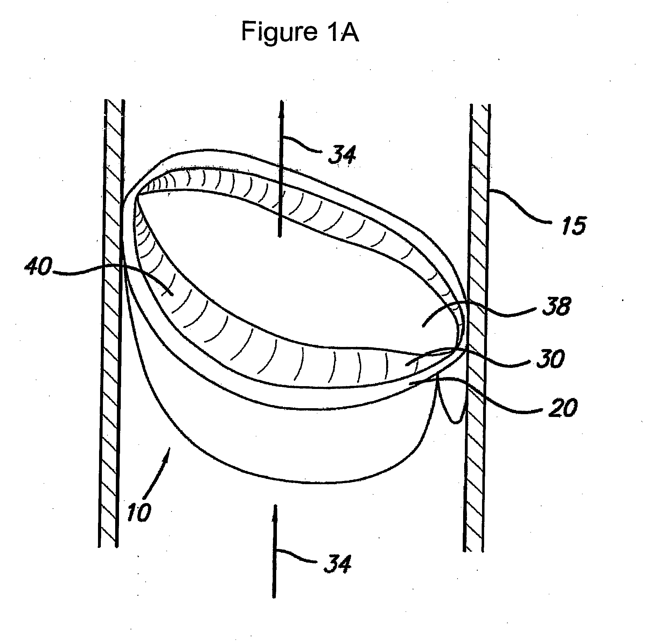

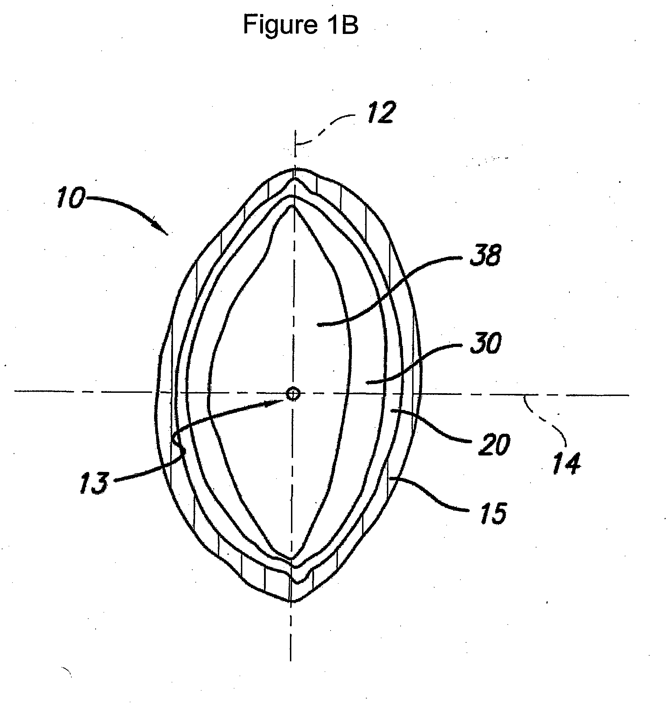

**FIG. 1B** is an alternative view of the embodiment shown in **FIG. 1A** with an open valve orifice;

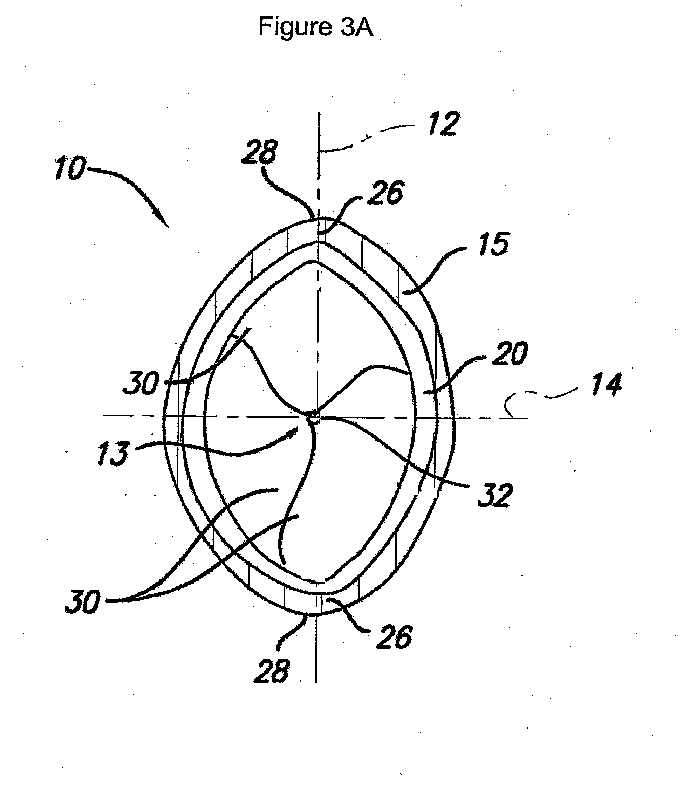

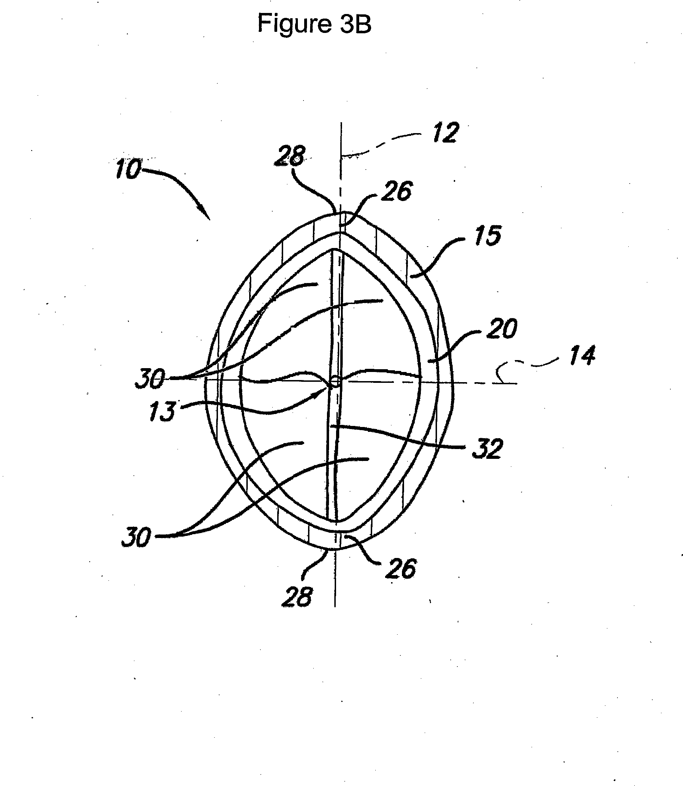

**FIGS. 3A** and **3B** are top views of elliptical valve devices having different numbers of leaflet leaflets;

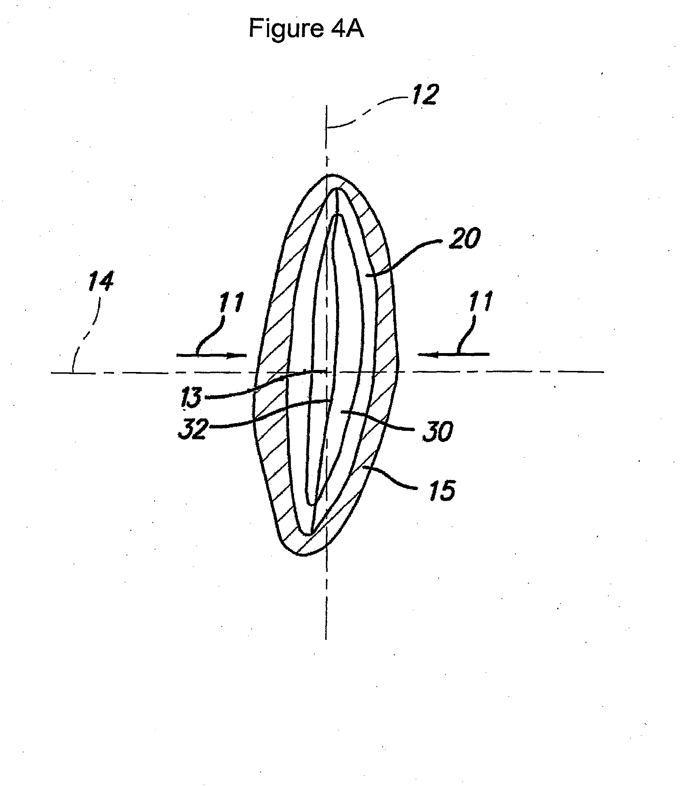

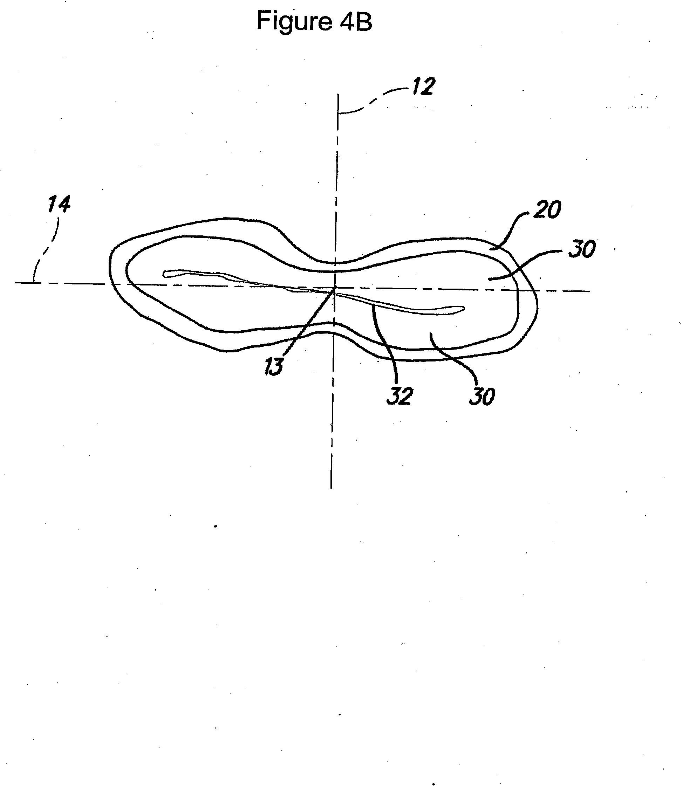

**FIG. 4A** is a top view of an elliptical valve device embodiment in a collapsed configuration along a first radial axis; FIG. **4B** is a top view of an elliptical valve device embodiment in a collapsed configuration along a second radial axis;

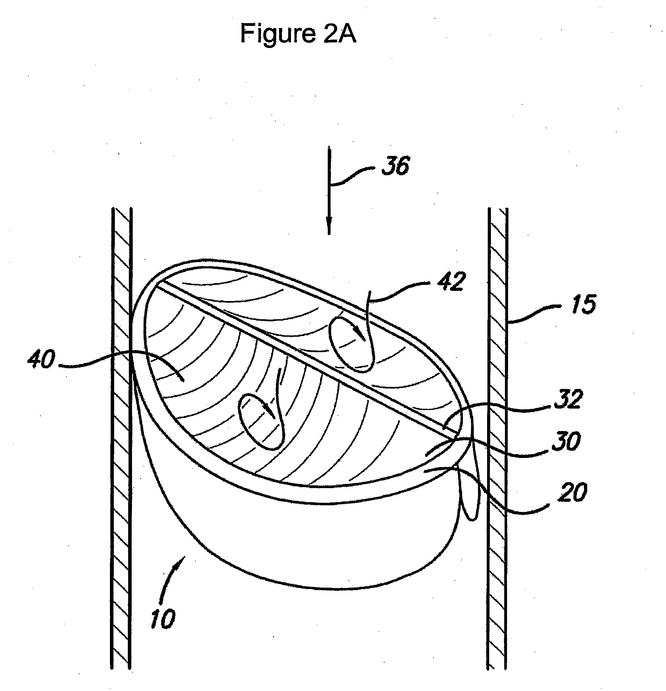

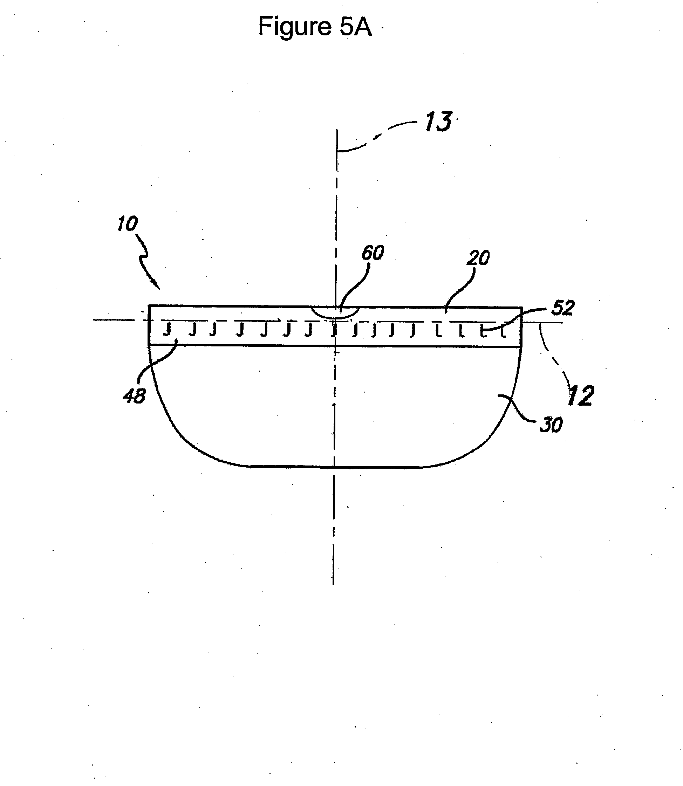

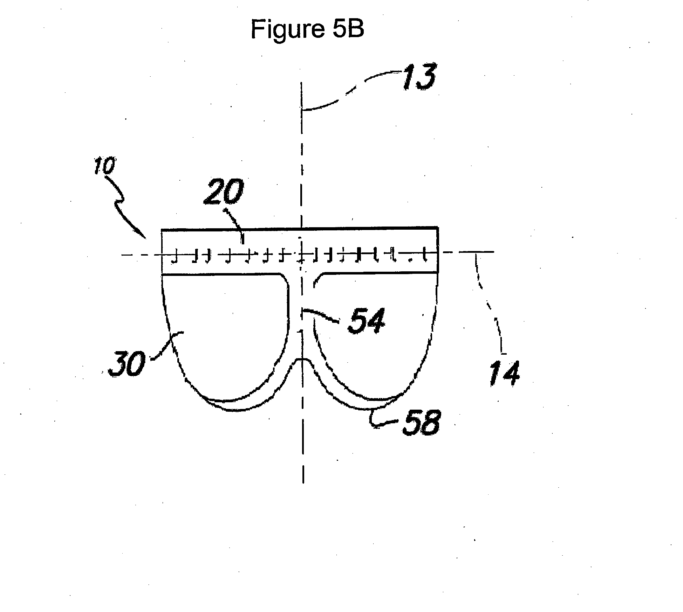

**FIG. 5A** is a first side view of the elliptical valve device embodiment shown in **FIG. 2A** in a closed configuration; **FIG. 5B** is a second side view of the elliptical valve device embodiment shown in **FIG. 2A****;**

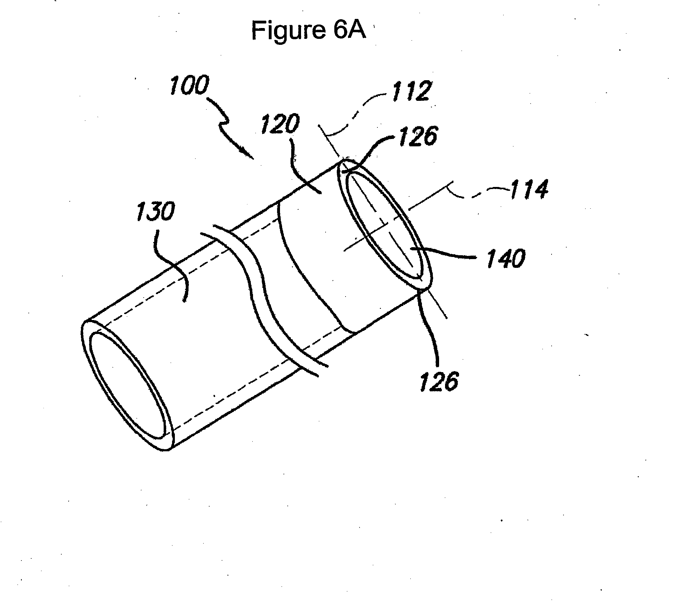

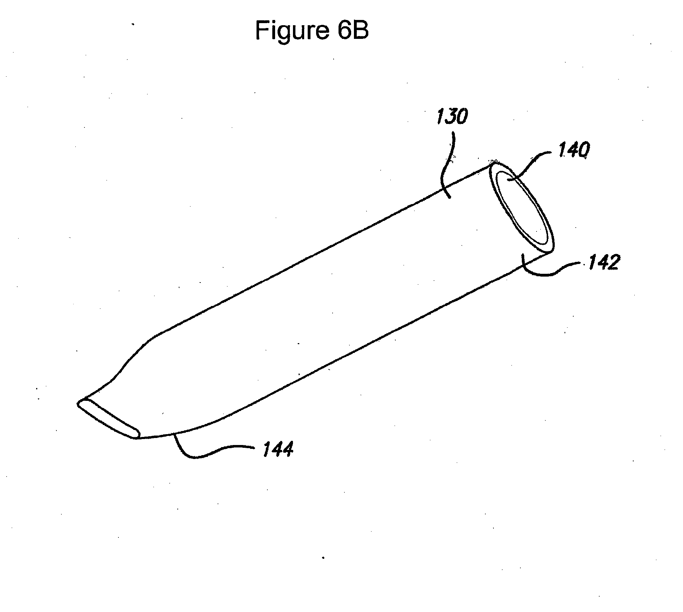

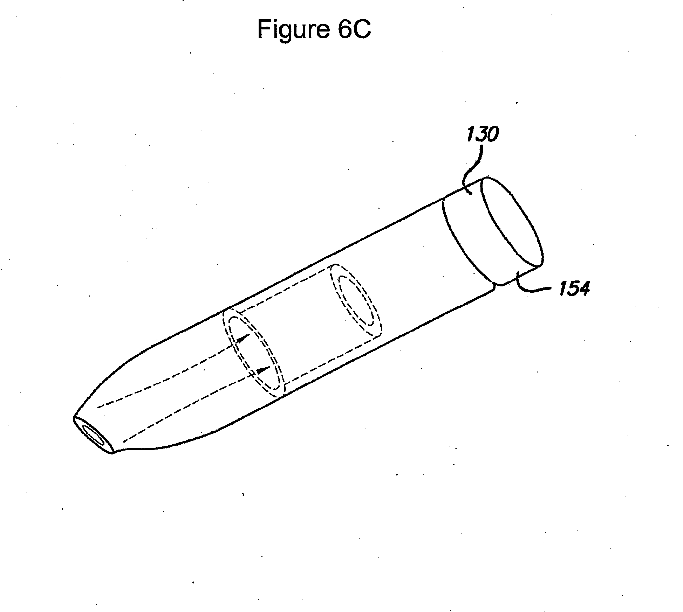

**FIG. 6A** is a cut-away perspective view of a flexible member of a frameless valve embodiment; **FIG. 6B** is a perspective view of the flexible member shown in **FIG. 6A** having a modified second end; and **FIG. 6C** is a perspective view of the embodiment shown in **FIG. 6B** having an inverted second end; and

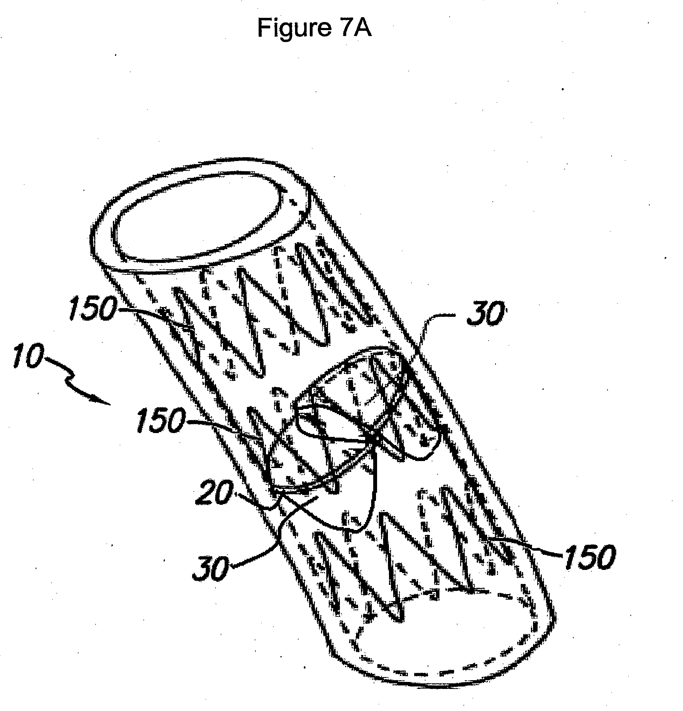

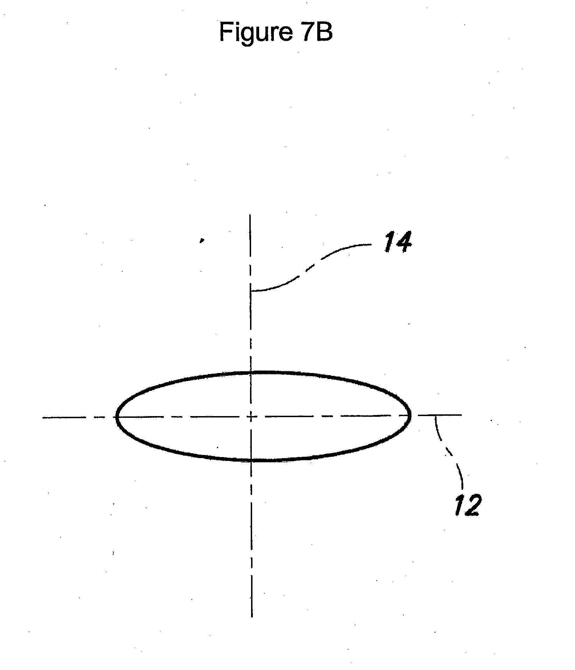

**FIG. 7A** is perspective view of an elliptical valve device embodiment of the present invention comprising an elliptical support; **FIG. 7B** is a cross section of the embodiment shown in **FIG. 7A****.**

### DETAILED DESCRIPTION

As described herein, an elliptical prosthetic valve device is provided for implantation within a body site having fluid flow. The valves of the present invention are suitable for implantation into vessels. The following detailed description and appended drawings describe and illustrate various exemplary embodiments of the invention. The description and drawings serve to enable one skilled in the art to make and use the invention.

As used herein, the term "implantable" refers to an ability of a medical device to be positioned at a location within a body, such as within a body vessel. Furthermore, the terms "implantation" and "implanted" refer to the positioning of a medical device at a location within a body, such as within a body vessel.

As used herein, the term "body vessel" means any body passage lumen that conducts fluid, including but not limited to blood vessels, esophageal, intestinal, billiary, urethral and ureteral passages. Preferably, the valves of the present invention are suitable for implantation into the vessels of the vasculature, such as veins, for regulating fluid flow through the vessel. The valves of the present invention may also be implanted in a passageway of the heart to regulate the fluid flow into and out of the heart. As used herein, the term "implantable" refers to an ability of a medical device to be positioned at a location within a body, such as within a body vessel, either temporarily, semi-permanently, or permanently. Permanent fixation of the valve device in a particular position is not required. Furthermore, the terms "implantation" and "implanted" refer to the positioning of a medical device at a location within a body, such as within a body vessel.

The terms "remodelable" or "bioremodelable" as used herein refer to the ability of a material to allow or induce host tissue growth, proliferation or regeneration following implantation of the tissue in vivo. Remodeling can occur in various microenvironments within a body, including without limitation soft tissue, a sphincter muscle region, body wall, tendon, ligament, bone and cardiovascular tissues. Upon implantation of a remodelable material, cellular infiltration and neovascularization are typically observed over a period of about 5 days to about 6 months or longer, as the remodelable material acts as a matrix for the ingrowth of adjacent tissue with site-specific structural and functional properties. The remodeling phenomenon which occurs in mammals following implantation of submucosal tissue includes rapid neovascularization and early mononuclear cell accumulation. Mesenchymal and epithelial cell proliferation and differentiation are typically observed by one week after in vivo implantation and extensive deposition of new extracellular matrix occurs almost immediately.

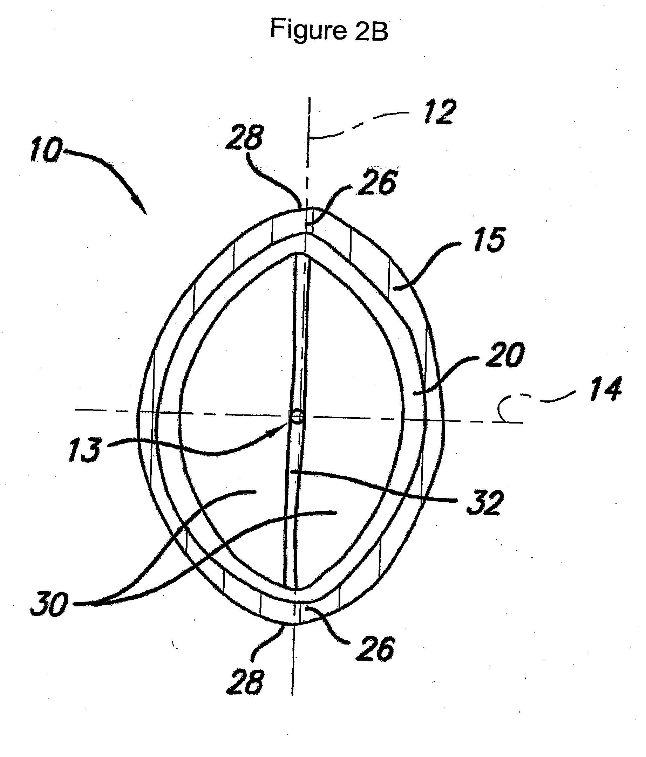

**FIG. 1A** is a perspective view of a first elliptical prosthetic valve device **10** comprising an elliptical support structure **20** configured as a substantially planar flexible ring attached to a pair of symmetrical valve leaflets **30.** **FIG. 1B** is a top view of the first elliptical prosthetic valve device **10** shown in **FIG. 1A****,** showing a first radial axis **12,** a second radial axis **14** of the elliptical support structure **20** in a plane perpendicular to a longitudinal axis **13.** **FIG. 2A** shows a perspective view of the first elliptical prosthetic valve device **10** of **FIGS. 1A-1B** in a closed valve configuration. **FIG. 2B** is a top view of the first elliptical prosthetic valve device **10** in the closed valve configuration shown in **FIG. 2A****,** showing a first radial axis **12,** a second radial axis **14 of** the elliptical support structure **20** in a plane perpendicular to a longitudinal axis **13.** **FIG. 5A** is a first side view of the first elliptical prosthetic valve device **10** shown in **FIGS. 2A-2B** showing the first radial axis **12** perpendicular to a longitudinal axis **13.** **FIG. 5B** is a second side view of the first elliptical prosthetic valve device **10** shown in **FIGS. 2A-2B** and **FIG. 5A** showing the second radial axis **12** perpendicular to a longitudinal axis **13.** The second side view of **FIG. 5B** is obtained by rotating the first elliptical prosthetic valve device **10** shown in the first side view of **FIG. 5A** 90-degrees around the longitudinal axis **13.**

**FIG. 1B** and **FIG. 2B** are top end views of an elliptical prosthetic valve device **10** embodiment implanted inside a portion of a body vessel **15.** **FIG. 1A** and **FIG. 2A** are side views of the elliptical prosthetic valve device **10** shown in **FIG. 1B** and **FIG. 2B**, respectively. The elliptical prosthetic valve device **10** can be implanted into an elliptical vessel **15**, such as a vein. The elliptical valve **10** is depicted with respect to a first radial axis **12** and a second radial axis **14,** both intersecting a longitudinal axis **13** of the valve. The second radial axis **14** is oriented perpendicular to the first radial axis **12** and in the same plane as the first radial axis **12.** The longitudinal axis **13** is oriented perpendicular to the first radial axis **12** and the second radial axis **14.** The prosthetic valve device **10** includes an elliptical support means configured as an elliptical support structure **20** having an outer surface with a substantially elliptical overall cross-sectional shape.

The elliptical support means can be formed from any suitable material that provides an elliptical cross sectional shape to the outer surface, while providing a desired amount of flexibility and resiliency. For example, the elliptical support means can be configured as an annular ring, a metal support frame, a molded polymer conduit, a rolled or reinforced portion of material, a woven section of material, an implantable frame having any suitable structure, or any combination thereof. Other materials suitable for forming the elliptical support means include biodegradable polymers, metals including metal alloys, biostable polymers, tissue or tissue components such as extracellular matrix materials, or biologically derived materials such as collagens. Preferably, the elliptical support means comprises a molded biocompatible thermoplastic polymer. The elliptical support structure **20** can have any suitable length and preferably defines a tubular interior lumen forming a conduit for fluid flow there through. The interior lumen can have any suitable cross-sectional shape, but preferably has an elliptical cross-sectional shape. Preferably, the lumen extends along a longitudinal axis **13** of the frame (perpendicular to the plane of the page), which perpendicularly intersects both the first radial axis **12** and the second radial axis **14.** The elliptical support structure **20** is depicted as a substantially planar, flexible ring structure bisected by a plane containing the first radial axis **12** and the second radial axis **14.**

The radial distance from the longitudinal axis **13** to the point where the outer surface of the elliptical support structure **20** intersects the first axis **12** is greater than the distance from the longitudinal axis **13** to the point where the outer surface of the elliptical support structure **20** intersects the second axis **14.** The elliptical support structure **20** also defines an interior lumen having a substantially elliptical cross sectional shape. The elliptical valve **10** preferably maintains an elliptical shape, even when fully expanded. Preferably, the elliptical cross-sectional shape of the elliptical support structure **20** conforms to an elliptical shape of the vessel into which the elliptical valve device **10** is implanted.

An elliptical support means can be designed to provide a desired level of flexibility in response to external force exerted radially inward on the elliptical support. For example, an elliptical support can be rigid or flexible in response to changes in the shape of a body vessel upon implantation. Incorporation of collapse points in the elliptical support can increase the flexibility of the elliptical support. For example, positioning pairs of collapse points in an elliptical support can increase the flexibility of the elliptical support along a first radial direction without substantially changing the flexibility in a second radial direction. Increased flexibility of an elliptical support is desirable, for example, change the shape of the elliptical support in response to changes in fluid flow or body vessel constriction or expansion.

The elliptical support means of the invention includes collapse points. Referring to **the valve 10 in** **FIG. 2B****,** collapse points **26** facilitate collapsing of the valve **10,** for example away from a symmetry plane containing the first radial axis **12** and second radial axis **14,** and toward a plane containing the first radial axis **12** and the longitudinal axis **13** (i.e., folding the frame "out of the page" or "into the page"). The collapse points **26** may be formed by any suitable method that provides a desired increase in the flexibility of a portion of the elliptical support or a support frame, for example by providing a reduced-thickness region as compared to the remainder of the elliptical support structure **20,** by providing a hinge, a gap or weak portion in the frame, or other equivalent structures as will be understood by one of skill in the art. Referring again to **FIG. 2B**, the elliptical support structure **20** comprises a pair of collapse points **26** along the first axis **12,** so that the elliptical support structure **20** is more flexible in response to radially inward force directed along the second axis **14,** compared to radially inward force along the first axis **12.** The elliptical support structure **20** may be collapsible along the first axis **12** as shown in **FIG. 4A** or along the second axis **14** as shown in **FIG. 4****B.** As shown in **FIG. 2B**, the collapse points **26** may be located at points of intersection **28** of the elliptical support structure **20** along the first axis **12** to provide an elliptical support structure **20** that collapses toward a first symmetry plane containing both the first radial axis **12** and the longitudinal axis **13.** Alternatively (and not within the scope of the claims), the collapse points **26** may be positioned at points of intersection of the second radial axis **14** with the elliptical support structure **20** to provide an elliptical support structure **20** that collapses toward a second symmetry plane containing both the second radial axis **14** and the longitudinal axis **13.** **FIG. 4A** shows the elliptical support structure **20** in a compressed state in response to radially inward force **11** applied along the second axis **14.** Alternatively, the collapse points **26** may be located along the second axis **14** when it is desirable for the elliptical support structure **20** to collapse along the second axis 14. **FIG. 4B** shows the elliptical support structure **20** in a compressed state in response to radially inward force applied along the first axis **12.** Any number of collapse points **26** may be located on the elliptical support structure **20.** The elliptical support structure **20** may also be collapsible along additional axes in response to changes in fluid flow or vessel constriction or expansion as will be understood by one of skill in the art.

The collapse points **26** may be formed by a hinge in the elliptical support means, or by a weakened portion of the support means. The relative weakness and strength of the various collapse points can be obtained in a variety of ways. For example, it may be possible to selectively treat a portion of the elliptical support means with heat, radiation, mechanical working, or combinations thereof, so that the mechanical characteristics of the hinge region are altered, i.e., so that selected hinge regions will bend, crack or break with a greater or lesser force than others of the hinge regions. In one aspect, the strength of the collapse points can be controlled by selecting the relative cross-sectional dimensions of the different regions of the elliptical support means. Usually, the collapse points will have cross-sectional dimensions which are selected so that the force required to bend, crack or sever the collapse point is less than that required for other non-collapse points. Usually, the collapse point will have a section in which the height in the radial direction remains constant (i.e. it will be the same as the remainder of the elliptical support means) while the width in the circumferential direction will be reduced about 20- 30% relative to the non-weakened hinge regions. The terms "weakened" and "non-weakened" are relative terms, and it would be possible to augment or increase the width of the non-weakened regions relative to the weakened regions. It will also be possible to provide two or more discrete narrowings within a single collapse point, or to provide one or more narrowings in the regions of the struts immediately adjacent to the collapse points. In another aspect, a collapse point may be created by cutting notches or voids into a portion of the elliptical support means. For example, V-shaped notches may be cut into the hinge region on the side which undergoes compression during opening of the hinge. Alternatively, the elliptical support means can be sanded or beveled to create a collapse point.

Preferably, the prosthetic valve also comprises a means for regulating fluid flow in a body vessel. The means for regulating fluid flow comprises a valve orifice having an open and a closed configuration, where the open configuration permits fluid flow through the body vessel in a first direction and the closed configuration substantially prevents fluid flow in the opposite direction. The means for regulating fluid flow can be one or more leaflets. Preferably, a leaflet comprises a free edge defining a portion of a valve orifice, and the free edge is moveable in response to fluid flow contacting the leaflet within a body vessel.

The device **10** shown in **FIGS. 1A-1B****,** **FIGS. 2A-2B** and **FIGS. 5A-5B** also includes a pair of leaflets **30** operably connected to the elliptical support structure **20.** The device **10** is shown in operation in **FIG. 1A** and **FIG. 2A****.** The two leaflets **30** operate to regulate fluid flow through the valve device **10** by allowing fluid flow in a first direction **34,** and substantially preventing fluid flow in a second, generally opposite direction **36** as illustrated in **FIGS. 6** and **7****,** respectively. **FIG. 1A** and **FIG. 1B** illustrate the **device 10** with an open valve orifice to permit fluid flow in a first direction **34** through an open valve orifice **38** defined by a free edge of each of the pair of leaflets **30.** When fluid flows through the body vessel **15** in the opposite direction **36,** the valve orifice **38** closes, as shown in **FIG. 2A** and **FIG. 2****B.**

As shown in **FIGS. 1A-1B****,** **FIGS. 2A-2B** and **FIGS. 5A-5B****,** a pair of leaflets **30** is connected to the elliptical support structure **20.** One of skill in the art will understand that the valve device **10** may include one leaflet, or a plurality of leaflets as illustrated in **FIGS. 3A-3B****,** such as two, three **(****FIG. 3A****),** four **(****FIG. 3B**), five or more leaflets. When two or more leaflets **30** are connected to the elliptical support structure **20,** the leaflets **30** meet to form a leaflet contact area **32.** The leaflet contact area **32,** formed when the valve orifice **38** is closed **(****FIGS. 2A-2B****,** **FIGS. 3A-3B****,** and **FIGS. 5A-5B****)** comprises a portion along the valve device **10** in which the facing surfaces of leaflets **30** coapt or lie in close proximity to one another. Preferably, the leaflets **30** may be shaped and sized to provide a sufficient leaflet contact area **32** to decrease the amount of retrograde flow in the second direction **36** through the valve device **10**. Desirably, the amount of retrograde fluid flow is about 1 - 10%, and preferably about 5-7% of the antegrade fluid flow. Preferably, the leaflets **30** are configured to maximize the leaflet contact area **32**, for example, by lengthening the leaflets **30** longitudinally with respect to the diameter of the vessel **15** into which the valve device **10** is implanted. By extending the leaflet contact area **32,** the valve device **10** can be configured to substantially seal during retrograde flow in the direction **36** so that undesired retrograde flow through the valve device **10** may be minimized. Prosthetic valves with smaller leaflet contact areas **32** may compromise the ability of valve leaflets **30** to sealably engage one another and, hence, for the prosthetic valve to seal during retrograde flow. Valve leaflets **30** connected to the elliptical support structure **20** may also contact the elliptical support structure **20** or the vessel **15** to regulate the fluid flow though the valve **10.**

As shown in FIGS. **1A** and **1B****,** the valve leaflets **30** connect to the elliptical support structure **20** to form a sealing engagement such that fluid substantially flows through a valve orifice **38** formed in the valve device **10** when the fluid flows in the first direction **34.** In some embodiments, the elliptical support structure **20** and the leaflets **30** may be formed together from the same material. When the elliptical support structure **20** is formed separately from the leaflets **30,** the leaflets **30** may be secured to the elliptical support structure **20** by any suitable means, including sewing, adhering, heat sealing, tissue welding, weaving, cross-linking, or otherwise suitable means for joining the leaflets **30** to the elliptical support structure **20.** As shown in **FIGS. 1** and **2A****,** the leaflets **30** may preferably be in the shape of pocket-forming receptacles and together with the elliptical support structure **20** form valve pockets **40** similar to natural sinuses formed by native valves. A natural sinus includes a distension of the vein wall, while a valve pocket **40** typically does not distend the vein wall. The valve pockets **40** substantially prevent fluid flow in the second direction **36** by trapping fluid flow between the leaflets **30** and the vessel wall **15** and the fluid in the valve pockets **40** pushes the leaflets **30** together to coapt at the contact area **32** and away from the vessel wall **15** to close the valve orifice **38** in the valve device **10.** The valve pockets **40** may be configured to allow the formation of fluid flow vortices **42** to prevent fluid from pooling or stagnating in the valve pockets 40. Stagnation of the fluid in the valve pockets **40** may lead to thrombosis or other problems. The leaflets **30** are desirably sized and shaped to provide sufficient coaptation and to minimize stagnation of fluid flow in the valve pockets 40. When fluid flow is in the first direction **34,** the leaflets **30** move toward the vessel wall **15** and fluid within the valve pockets 40 is expelled from the valve pockets **40** as the leaflets **30** move toward the vessel wall **15** as shown in **FIG. 1A****.**

A first side view of the elliptical valve device **10** is shown in **FIG. 5A**. The leaflets **30** are connected to the elliptical support structure **20.** An attachment portion **48** is shown operably connected to the elliptical support structure **20** for attaching the elliptical valve device **10** to the vessel wall **15** in any suitable manner. Exemplary techniques for attachment include vessel engaging features, such as barbs or hooks, suturing, stapling, bonding, gluing or otherwise adhering the device **10** to a vessel wall, or combinations thereof. The attachment portion **48** may include bioresorbable sealants and adhesives to secure the valve device **10** to the vessel wall **15.** Examples of bioresorbable sealants and adhesives include FOCALSEAL^{®} (biodegradable eosin-PEG-lactide hydrogel requiring photopolymerization with Xenon light wand) produced by Focal; BERIPLAST^{®} produced by Adventis-Bering; VIVOSTAT^{®} produced by ConvaTec (Bristol-Meyers-Squibb); SEALAGEN™ produced by Baxter; FIBRX^{®} (containing virally inactivated human fibrinogen and inhibited-human thrombin) produced by CryoLife; TISSEEL^{®} (fibrin glue composed of plasma derivatives from the last stages in the natural coagulation pathway where soluble fibrinogen is converted into a solid fibrin) and TISSUCOL^{®} produced by Baxter; QUIXIL^{®} (Biological Active Component and Thrombin) produced by Omrix Biopharm; a PEG-collagen conjugate produced by Cohesion (Collagen); HYSTOACRYL^{®} BLUE (ENBUCRILATE) (cyanoacrylate) produced by Davis & Geck; NEXACRYL™ (N-butyl cyanoacrylate), NEXABOND™, NEXABOND™ S/C, and TRAUMASEAL™ (product based on cyanoacrylate) produced by Closure Medical (TriPoint Medical); DERMABOND™ which consists of 2-Octyl Cyanoacrylate produced by Dermabond (Ethicon); TISSUEGLU^{®} produced by Medi-West Pharma; and VETBOND™ which consists of n-butyl cyanoacrylate produced by 3M.

Alternatively, or in addition to, adhesives and sealants, the attachment portion **48** may comprise one or more structures for anchoring the medical device, such as a plurality of barbs. As shown in **FIG. 5A****,** individual barbs **52** are provided. The barbs **52** may be formed from a portion of the attachment portion, an elliptical support structure, or a frame, or may be formed from separate structures individually secured to the elliptical support structure **20** by any means known to one of skill in the art, including but not limited to stitching and adhesive. The barbs **52** may be provided along a wire element, with each barb **52** being spaced apart along the wire element secured to the elliptical support structure **20** (not shown). The wire element itself, for barb attachment, preferably does not serve to exert radial force upon the vessel wall to retain the position of the device, as would a stent.

As shown in **FIG. 5B**, a second side view of the first valve device 10 shows a reinforcing portion **54** connected to the elliptical support along the second axis **14** for shaping or support of the valve device **10.** The second side view is obtained by rotating the valve device **10** view of **FIG. 5A** by 90 degrees around the longitudinal axis **13.** The reinforcing portion **54** may be formed from the same material as the valve leaflet, for example by increasing the number of layers of material, by treating the leaflet material, or otherwise strengthening a portion **58** of the leaflets **30.** Alternatively or additionally, the reinforcing portion **54** may be formed from the elliptical support material and form an extension thereof. The materials for the leaflets **30** and the elliptical support structure **20** will be discussed below. The reinforcing portion **54** may be collapsible so as not to interfere with the collapsibility of the elliptical support structure **20** as described above. The reinforcing portion **54** bridges the points of intersection of the elliptical ring with the first radial axis (e.g., collapse points **26)** and is symmetrically bisected by a plane containing the longitudinal axis and first radial axis. The reinforcing portion **54** can be configured as an arch joining portions of an elliptical support structure **20.**

Another embodiment of the present invention is shown **FIGS. 6A**-6C where a second elliptical valve device **100** includes a tubular-shaped flexible member **130** connected to an elliptical support **120**. The elliptical support **120** is substantially similar to the elliptical support structure **20** described above and includes a first radial axis **112** and a second radial axis **114** extending perpendicular to a longitudinal axis **113.** The valve device **100** further includes an attachment portion **154,** similar to the attachment portion **54** described above, configured to secure the valve device **100** to a body vessel in a manner. The elliptical support **120** may further include one or more collapse points **126** to facilitate collapsing of the valve **100.**

The flexible member **130** is adapted to regulate fluid flow through a lumen **140** extending longitudinally through the valve device **100.** The flexible member **130** conforms to the elliptically shaped elliptical support **120** to form an elliptical valve device **100** that is readily collapsible in the implantation site. As shown in **FIG**. **6B**, the flexible member **120** includes a first end **142** and a second end **144** having the lumen **140** formed in the flexible member **120** between the first end **142** and the second end **144.** The lumen **140** may be any chamber, channel, opening, bore, orifice, flow passage, passageway, or cavity. The inner diameter of the lumen need not be constant. For example, the flexible member **120** may include a sinus (not shown) similar to a native sinus where there is a bulging or bowing of the lumen.

To reverse the direction of fluid flow through the lumen **140** of the flexible member **130,** the second end **144** of the flexible member **130** may be inverted into the lumen **140.** The inverted portion of the flexible member may be secured to itself by any suitable means including adhesives, tissue welding, wires, crimping, bands, chemical cross-linking, heating, light, including laser, radiofrequency, and sewing. **FIGS. 6A-6C** show inversion of the second end **144** of the flexible member **130** into itself. **FIG. 6A** shows the flexible member **130** prior to inversion. **FIG. 6B** shows an embodiment where the second end **144** may be modified prior to inversion, such as by narrowing. **FIG. 6C** shows the second end **144** after inversion into the flexible member **130.** Inversion of the flexible member **130** includes infolding (e.g., tucked, folded inward, turned outside in, rolled inward, folded toward the inside of the tubular structure, inverted into the lumen, inserted into the lumen, or otherwise gathering and moving materials in these described directions. The valve device **100** may also include any additional features described herein with reference to the valve device **10.** Exemplary tubular flexible members and methods of making such members may be found in U.S. Application Serial No. 10/909,153.

In some embodiments, an elliptical support structure **20, 120** may be formed from a porous material that encourages tissue ingrowth. For example, the support material may be formed from a porous biocompatible material, such as a biocompatible polyurethane, polytetrafluoroethylene, expanded polytetrafluoroethylene, or a porous extracellular matrix material, such as small intestine submucosa (SIS), mesh to encourage tissue ingrowth into portions of the elliptical valve. SIS may also be attached to a mesh to form the elliptical support structure 20 or a portion thereof. The leaflets **30, 130** may be formed from a synthetic material such as the biocompatible polyurethane sold under the tradename THORALON^{®}. The leaflets **30, 130** can be connected to the elliptical support structure **20,120.**

The elliptical valve device 10, 100 may further include a radiopaque material to form an imageable element for orienting the valve within a body vessel lumen. The radiopaque material can be identified by remote imaging methods including X-ray, ultrasound, Magnetic Resonance Imaging, fluoroscope and the like, or by detecting a signal from or corresponding to the marker. An elliptical valve can include radiopaque indicia to provide information relating to the orientation of the valve within the body vessel. A valve or delivery device may comprise one or more radiopaque materials to facilitate tracking and positioning of the valve, which may be added in any fabrication method or absorbed into or sprayed onto the surface of part or all of the valve. For example, radiopaque markers can be used to identify a long axis or a short axis of a medical device within a body vessel. Radiopaque material may be attached to an elliptical support structure or woven into portions of the valve leaflet material. The degree of radiopacity contrast can be altered by changing the composition of the radiopaque material. For example, radiopaque material may be covalently bound to the support member. Common radiopaque materials include barium sulfate, bismuth subcarbonate, and zirconium dioxide. Other radiopaque materials include: cadmium, tungsten, gold, tantalum, bismuth, platinum, iridium, iodine and rhodium. An exemplary imageable element **60** is shown in **FIG. 5A**. Exemplary prosthetic valve devices and imageable elements are further described in U.S. Publication No. 2004/0167619. Briefly, the imageable element may be placed anywhere on the valve **10, 100,** for example, but not limited to, the attachment portion, the elliptical support, the leaflets, the frame, a covering, and the like. The imageable element will allow the clinician to position the valve **10,100** in the vessel wall **15** in the desired orientation in the delivery device during implantation and monitor the position of the valve **10, 100** after implantation. Alternatively, the imageable element may be provided on a delivery device to facilitate the positioning of the valve **10, 100** in the vessel wall **15.** A single or multiple imageable elements may be included on the valve **10** or the delivery device to facilitate placement of the valve **10, 100.** The imageable element may be applied to the prosthetic valve **10,100** by any well known technique, including but not limited to, dipping, electrostatic deposition, spraying, painting, overlaying, wrapping and others. For example, a portion of the prosthetic valve **16** may be dipped in molten gold. Typically, an imageable material, such as gold metal, is configured as a rivet with a diameter of about 0.5 mm, can be punched into a portion of the elliptical support structure. The gold rivet can have. Optionally, a protective polymer overcoat may be applied to prevent degradation of the imaging material. A polymer resin coating may be applied to a portion of the valve **10** that includes radiopaque filler material such as barium sulfate, bismuth, or tungsten powder. Alternatively, the imageable element may be formed from radiopaque wire or thread including gold, platinum, titanium and the like that may be used to form a portion of the prosthetic valve **10, 100.** Preferably, the imageable element will not alter or interfere with the function of the valve **10,100.**

In some embodiments of the present invention, the elliptical support means may include a support frame. Referring to **FIG**. **7A****,** a third elliptical Valve **10'** comprises a support frame **150** for support and implantation of the elliptical valve device **10'** and will be described and shown with reference to the valve device **10'.** As shown in **FIG. 7A****,** the frame **150** extends from the elliptical support structure **20** and contacts the wall of the vessel **15.** The leaflet **30** extends from the elliptical support structure **20.** **FIG. 7B** is a cross sectional view of the elliptical support structure **20.** Any suitable implantable frame can be used as the support frame **150** in the elliptical valve **10'.** The specific support frame chosen will depend on several considerations, including the size and configuration of the vessel at the implantation site and the size and nature of the valve device **10.** A support frame that provides a stenting function, i.e., exerts a radially outward force on the interior of the body vessel in which the elliptical valve device **10** is implanted, may also be included. By including a support frame that provides a stenting function, the elliptical valve device **10'** can provide a stenting functionality at a point of treatment in a body vessel. The stent art provides numerous examples of support frames acceptable for use with the elliptical valve device **10',** and any suitable stent can be used as the support frame **150.** Exemplary configurations for the support frame **150** include, but are not limited to, braided strands, helically wound strands, ring members, consecutively attached ring members, tube members, and frames cut from solid tubes. If a stent is used as the support frame **150,** the specific stent chosen will depend on several factors, including the vessel into which the valve device is being implanted, the axial length of the treatment site, the number of valves desired in the device, the inner diameter of the body vessel, the delivery method for placing the support frame, and others. Those skilled in the art can determine an appropriate stent based on these and other factors.

The illustrated support frame **150** is an expandable support frame comprising a plurality of interconnected struts, and having radially compressed and radially expanded configurations, allowing the elliptical valve device **10'** to be delivered to and implanted at a point of treatment using percutaneous techniques and devices. The support frame **150** can be self-expandable. In some embodiments, the self-expanding support frame **150** can be compressed into a low-profile delivery conformation and then constrained within a delivery system for delivery to a point of treatment in the lumen of a body vessel. At the point of treatment, the self-expanding support frame **150** can be released and allowed to subsequently expand to another configuration.

The support frame can have any suitable size. The exact configuration and size chosen will depend on several factors, including the desired delivery technique, the nature of the body vessel in which the valve device **10'** will be implanted, and the size of the vessel. The support frame can be sized so that the second, expanded configuration is slightly larger in diameter that the inner diameter of the vessel in which the medical device will be implanted. This sizing can facilitate anchoring of the valve device **10'** within the vessel wall 15 and maintenance of the valve device **10'** at a point of treatment following implantation. Examples of suitable support frames' 150 for use in the valve device of the present invention include those described in U.S. Patent Nos. 6,508,833; 6,464,720; 6,231,598; 6,299,635; 4,580,568; and U.S. Patent Application Publication No. 2004/018658 A1, U.S. Application Serial No. 11/099,713, filed April 6, 2005.

The elliptical valve device of the present invention may be delivered to a lumen of a body vessel by various techniques known in the art and will be described with reference to a valve device. By way of non-limiting example, the valve device may be delivered and positioned in the body vessel using a catheter. For delivery, the valve device may be placed in an unexpanded configuration to fit in the lumen of a delivery catheter. The catheter is then introduced into the body vessel and its tip positioned at a point of treatment within the body vessel. The valve device may then be expelled from the tip of the catheter at the point of treatment. Once expelled from the catheter, the valve device may expand to the expanded configuration and engage the interior wall of the body vessel, preferably using attachment portion provided on the valve device. The valve device may be self-expanding or expandable by a balloon of a balloon catheter as will be understood by one of skill in the art. Delivery has been described using a delivery catheter as an example, the valve device may be delivered to a position within a body by any means known to one of skill in the art. Exemplary delivery devices suitable for implanting the valve include U.S. Publication Nos. 2004/0225344 and 2003/0144670.

The elliptical valve device may be made from a variety of materials known to one of skill in the art. The valve device may be made from a single material or a combination of materials. Desirably, the medical device is constructed from materials that are both compatible with all fluids of a mammalian body, i.e., when implanted in the body of a mammal, the materials are biologically inert or interact with bodily fluids to become biologically inert, physiologically acceptable, non-toxic, and insoluble. The materials from which the heart valve is constructed are typically naturally derived or based on a synthetic biocompatible organic polymer. The material or materials need only be biocompatible or able to be rendered biocompatible. The term "biocompatible" refers to a material that is substantially non-toxic in the *in vivo* environment of its intended use, and that is not substantially rejected by the patient's physiological system (i.e., is non-antigenic). This can be gauged by the ability of a material to pass the biocompatibility tests set forth in International Standards Organization (ISO) Standard No. 10993 and/or the U.S. Pharmacopeia (USP) 23 and/or the U.S. Food and Drug Administration (FDA) blue book memorandum No. G95-1, entitled "Use of International Standard ISO-10993, Biological Evaluation of Medical Devices Part-1: Evaluation and Testing." Typically, these tests measure a material's toxicity, infectivity, pyrogenicity, irritation potential, reactivity, hemolytic activity, carcinogenicity and/or immunogenicity. A biocompatible structure or material, when introduced into a majority of patients, will not cause a significantly adverse, long-lived or escalating biological reaction or response, and is distinguished from a mild, transient inflammation which typically accompanies surgery or implantation of foreign objects into a living organism.

Preferably, the elliptical support means is formed from a flexibly resilient material, e.g., a thermoplastic elastomeric polymer such as a suitable polyurethane material, such as a silicone-polyurethane co-polymer. Synthetic biocompatible organic polymers which can be used to form the elliptical support include, but are not limited to, siloxane polymers, polydimethylsiloxanes, silicone rubbers, polyurethane, polyether urethane, polyetherurethane urea, polyesterurethane, polyamide, polycarbonate, polyester, polypropylene, polyethylene, polystyrene, polyvinyl chloride, polytetrafluoroethylene, polysulfone, cellulose acetate, polymethylmethacrylate, and poly(ethylene/vinylacetate). Natural materials from which the elliptical support can be constructed include bovine pericardium tissue and porcine tissue, among others. In one embodiment, the elliptical support is constructed from a high performance silicone rubber, such as a platinum-catalyzed silicone elastomer made from dimethylsiloxane, as is known by the tradename HP-100 (Dow Corning, Midland, Mich.; an alternate Dow Coming product code for this product is X7-4978). Other silicone rubber polymers may be used.

Any suitable portion of the elliptical valve device, including, but not limited to, the elliptical support structure, the leaflets, a flexible member, an attachment portion, a collapse point and the support frame may comprise a bioabsorbable material that can be degraded and absorbed by the body over time to advantageously eliminate the portion formed from the bioabsorbable material from the vessel before, during or after a tissue remodeling process occurs at the implantation site. The first pair of collapse points comprise a bioabsorbable material. A number of bioabsorbable polymers, copolymers, or blends of bioabsorbable polymers can also be used, inclding polyesters such as poly-alpha hydroxy and poly-beta hydroxy polyesters, polycaprolactone, polyglycolic acid, polyether-esters, poly(p-dioxanone), polyoxaesters; polyphosphazenes; polyanhydrides; polyethers including polyglycols polyorthoesters; expoxy polymers including polyethylene oxide; polysaccharides including cellulose, chitin, dextran, starch, hydroxyethyl starch, polygluconate, hyaluronic acid; polyamides including polyamino acids, polyester-amides, polyglutamic acid, polylysine, gelatin, fibrin, fibrinogen, casein, and collagen. Other examples of biocompatible homo- or co-polymers suitable for use in the present invention include vinyl polymers including polyfumarate, polyvinylpyrolidone, polyvinyl alcohol, poly-N-(2-hydroxypropyl)-methacrylamide, polyacrylates, and polyalkylene oxalates.

In certain embodiments of the invention, at least a portion of the valve material may be comprised of a naturally derived or synthetic collagenous material, for instance, an extracellular matrix material. Suitable extracellular matrix materials include, for instance, submucosa (including, for example, small intestine submucosa (SIS), stomach submucosa, urinary bladder submucosa, or uterine submucosa), renal capsule membrane, dura mater, pericardium, serosa, peritoneum or basement membrane materials, including liver basement membrane. Extracellular material (ECM) such as SIS or other types of submucosal-derived tissue may have a remodelable quality that can be used as scaffolding to induce the growth and proliferation of neurological related tissues and to serve as a matrix for the regrowth of native tissues over time, which tissue may be referred to as tissue derived from ECM or SIS, or may be cross linked to affect the degree of remodelability. The material used herein may be made thicker by making multilaminate constructs. These layers may be isolated and used as intact natural sheet forms, or reconstituted collagen layers including collagen derived from these materials or other collagenous materials may be used. For additional information as to submucosa materials useful in the present invention, and their isolation and treatment, reference can be made to U.S. Patent No. 6,206,931 and U.S. Patent Application Publication No. 2004/0180042. Whether the valve material is synthetic or naturally occurring, the graft member and leaflets can be made thicker by using a multilaminate construct, for example, SIS constructs as described in U.S. Patent Nos. 5,968,096; 5,955,110; 5,885,619. Composite materials comprising polymeric materials and tissue-derived extracellular matrix materials can also be used, including ePTFE-SIS composite materials.

In some embodiments, the valve leaflets 30 may be tissue leaflets. Tissue valves may be constructed with native tissues, for example, but not limited to, porcine valves and leaflets, or with separate leaflets cut from bovine pericardium. Any source for tissue leaflets known to one of skill in the art may be used for the leaflets of the present invention. In preferred embodiments, the valve has two or more leaftets, typically two.

In one aspect, the valve leaflet can be formed from cross-linked tissues, such as small intestine submucosa. Cross-linking can be performed, for example, to mechanically stabilize the material to the device. Cross-linked material generally refers to material that is completely cross-linked in the sense that further contact with a cross-linking agent does not further change measurable mechanical properties of the material. Cross-linking can be accomplished with lyopholization, adhesives, pressure and or/heat. Chemical cross-linking can also be used to join layers of material together. Other cross-linking agents can incorporate glutaraldehyde, albumin, formaldehyde or a combination thereof. Material can also be fixed by cross-linking. Fixation provides mechanical stabilization, for example, by preventing enzymatic degradation of the tissue and by anchoring the collagen fibrils. Other cross-linking agents can be used to form cross-linking regions, such as epoxides, epoxyamines, diimides and other difunctional polyfunctional aldehydes. In particular, aldehyde functional groups are highly reactive with amine groups in proteins, such as collagen. Epoxyamines are molecules that generally include both an amine moiety (e.g. a primary, secondary, tertiary, or quaternary amine) and an epoxide moiety. The epoxyamine compound can be a monoepoxyamine compound and or a polyepoxyamine compound. In some embodiments, the epoxyamine compound is a polyepoxyamine compound having at least two epoxide moieties and possibly three or more epoxide moieties. In some embodiments, the polyepoxyamine compound is triglycidylamine (TGA). The use of cross-linking agents form corresponding adducts, such as glutaraldehyde adducts and epoxyamine adducts, of the cross-linking agent with the material that have an identifiable chemical structures.

If constructed with a polymer such as silicone rubber or modified polyetherurethane, the valve leaflets can be constructed as follows: the polymer is dissolved in a solvent, e.g. an amide such as dimethylacetamide (DMAC) or dimethylformamide (DMF) (for polyetherurethane), respectively. Other solvents may be employed without departing from the scope of the invention. Selection of suitable solvents for particular polymers is within the level of ordinary skill in the art. Typically, the polymer is dissolved to about 8-14% w/v, more preferably about 10% w/v, although this concentration can be varied as desired. After the polymer is dissolved, a stent is repeatedly dipped into the polymer solution and dried in air at about 15-25% relative humidity, preferably about 20% relative humidity. In addition to the dipping technique described herein, the valve may be formed by injection, transfer, or compression molding, thermoforming, or other techniques known in the art.

The elliptical support structure, support frame, and the attachment portion may be formed from the same material or different materials. Examples of suitable materials for the elliptical support structure, support frame, and the attachment portions, as well as other portions of the valve device include, without limitation, stainless steel (such as 316 stainless steel), nickel titanium (NiTi) alloys, e.g., Nitinol, other shape memory and/or superelastic materials, MP35N, gold, silver, a cobalt-chromium alloy, tantalum, platinum or platinum iridium, or other biocompatible metals and/or alloys such as carbon or carbon fiber, cellulose acetate, cellulose nitrate, silicone, cross-linked polyvinyl alcohol (PVA) hydrogel, cross-linked PVA hydrogel foam, styrene isobutylene-styrene block copolymer (Kraton), polyethylene terephthalate, polyurethane, polyamide, polyester, polyorthoester, polyanhydride, polyether sulfone, polycarbonate, polypropylene, high molecular weight polyethylene, polytetrafluoroethylene, or other biocompatible polymeric material, or mixture of copolymers thereof, or stainless steel, polymers, and any suitable composite material. For valves comprising support frames, the support frame material can also be a hard polymer, such as high durometer polyurethane, polyacetal, or another polymer with a high degree of stiffness, or metals such as cobalt-chromium alloy, titanium alloy or Nitinol, can be used.

Particularly preferred materials for self-expanding implantable frames are shape memory alloys that exhibit superelastic behavior, i.e., are capable of significant distortion without plastic deformation. Frames manufactured of such materials may be significantly compressed without permanent plastic deformation, i.e., they are compressed such that the maximum strain level in the stent is below the recoverable strain limit of the material. Discussions relating to nickel titanium alloys and other alloys that exhibit behaviors suitable for frames can be found in, e.g., U.S. Pat. No. 5,597,378 (Jervis) and WO 95/31945 (Burmeister et al.). A preferred shape memory alloy is Ni-Ti, although any of the other known shape memory alloys may be used as well. Such other alloys include: Au--Cd, Cu--Zn, In--Ti, Cu--Zn--Al, Ti--Nb, Au--Cu--Zn, Cu--Zn--Sn, CuZn--Si, Cu--Al--Ni, Ag--Cd, Cu--Sn, Cu--Zn--Ga, Ni--Al, Fe--Pt, U--Nb, Ti--Pd--Ni, Fe-Mn--Si, and the like. These alloys may also be doped with small amounts of other elements for various property modifications as may be desired and as is known in the art. Nickel titanium alloys suitable for use in manufacturing implantable frames can be obtained from, e.g., Memory Corp., Brookfield, Conn. One suitable material possessing desirable characteristics for self-expansion is Nitinol, a Nickel-Titanium alloy that can recover elastic deformations of up to 10 percent. This unusually large elastic range is commonly known as superelasticity.

In yet another preferred embodiment, a valve comprises a polyurethane material. For example, a valve leaflet can be formed from a suitable biocompatible material comprising polyurethane derivatives. An exemplary preferred polyurethane derivative is a polyetherurethane urea formerly available under the tradename Biomer (Ethicon Inc., Somerville, N.J.). In other embodiments, at least a portion of the valve device **10,10',100,** such as a valve leaflet, may be formed from a biocompatible modified polyetherurethane. Although preparation of an exemplary phosphonate-modified polyetherurethane, referred to herein as "F2000-HEDP," is described herein, the invention is not restricted to any particular polyetherurethane species. The base polyetherurethane (PEU F-2000) is synthesized from diphenylmethane-4,4'-diisocyanate (MDI), a 1,4- butanediol chain extender (BD), and a polytetramethylene oxide with a molecular weight of about 2000 (PTMO-2000) (available under the tradename Terethane 2000 Polyether Glycol, Dupont, Wilmington, Del.). The reactant ratio of MDI:BD:PTMO-2000 is 5:3:2, with 1.7% hydroxyl excess. The modified polyetherurethane is obtained by reacting, typically, ethanehydroxydiphosphonate (HEDP, available from Monsanto Company, St. Louis, Mo., as Dequest 2010) with a polyfunctional epoxide (such as Denacol 521, available from Nagasi Chemicals, Osaka, Japan), and then with the PEU F-2000 base polymer. (Details on the synthesis of F2000-HEDP are provided in U.S. Pat. No. 5,436,291, whose entire contents are hereby incorporated by reference herein.) The ratio of HEDP to total final polymer is typically about 100 to about 400 nmol/mg.

In a further embodiment, the elliptical support or a valve leaflet is constructed from a polyetherurethane/polysiliconeurethane. An exemplary preferred polyetherurethane/polysiliconeurethane may be referred to herein as "F2000/Dow Coming 7150," although other polyetherurethane/ polysiliconeurethanes can be used, such as F2000/Dow Coming 7150 comprising F2000 polyetherurethane, as described above, with a final coat of a polysiliconeurethane, such as formerly available as Dow Coming 7150, now available as Dow Coming X7- 4074.

One type of preferred biocompatible polyurethane material suitable for use in forming the valve device, including portions thereof such as valve leaflets, is sold under the tradename THORALON^{®} (THORATEC, Pleasanton, CA). THORALON^{®} is described in U.S. Pat. Application Publication No. 2002/0065552 A1 and U.S. Pat. No. 4,675,361, both of which are incorporated herein by reference in their entirety. THORALON^{®} is a polyurethane base polymer (referred to as BPS-215) blended with a siloxane containing surface modifying additive (referred to as SMA-300). The concentration of the surface modifying additive may be in the range of 0.5% to 5% by weight of the base polymer.

The SMA-300 component (THORATEC) is a polyurethane comprising polydimethylsiloxane as a soft segment and the reaction product of diphenylmethane diisocyanate (MDI) and 1,4-butanediol as a hard segment. A process for synthesizing SMA-300 is described, for example, in U.S. Pat. Nos. 4,861,830 and 4,675,361, which are incorporated herein by reference in their entirety. The BPS-215 component (THORATEC) is a segmented polyetherurethane urea containing a soft segment and a hard segment. The soft segment is made of polytetramethylene oxide (PTMO), and the hard segment is made from the reaction of 4,4'-diphenylmethane diisocyanate (MDI) and ethylene diamine (ED).

Polyurethane materials such as THORALON^{®} can be manipulated to provide either porous or non-porous THORALON^{®}. Porous THORALON^{®} can be formed by mixing the polyetherurethane urea (BPS-215), the surface modifying additive (SMA-300) and a particulate substance in a solvent. The particulate may be any of a variety of different particulates or pore forming agents, including inorganic salts. Preferably the particulate is insoluble in the solvent. The solvent may include dimethyl formamide (DMF), tetrahydrofuran (THF), dimethyacetamide (DMAC), dimethyl sulfoxide (DMSO), or mixtures thereof. The composition can contain from about up to about 40 wt% polymer, preferably up to about 5% to about 25%, and different levels of polymer within the range can be used to fine tune the viscosity needed for a given process. The composition can more preferably contain up to about 5 wt% polymer for some spray application embodiments and up to about 20% for applying the material to a mold surface or a mandrel by dipping. The soluble particulates can be mixed into the composition. For example, the mixing can be performed with a spinning blade mixer for about an hour under ambient pressure and in a temperature range of about 18 °C to about 27 °C. The entire composition can be cast as a sheet, or coated onto an article such as a mandrel or a mold. In one example, the composition can be dried to remove the solvent, and then the dried material can be soaked in distilled water to dissolve the particulates and leave pores in the material. In another example, the composition can be coagulated in a bath of distilled water. Since the polymer is insoluble in the water, it will rapidly solidify, trapping some or all of the particulates. The particulates can then dissolve from the polymer, leaving pores in the material. It may be desirable to use warm water for the extraction, for example water at a temperature of about 60 °C. The resulting pore diameter can also be substantially equal to the diameter of the salt grains.

The porous polymeric sheet can have a void-to-volume ration from about 0.20 to about 0.90. Preferably the void-to-volume ratio is from about 0.65 to about 0.80. The resulting void-to-volume ratio can be substantially equal to the ratio of salt volume to the volume of the polymer plus the salt. Void-to-volume ratio is defined as the volume of the pores divided by the total volume of the polymeric layer including the volume of the pores. The void-to-volume ratio can be measured using the protocol described in AAMI (Association for the Advancement of Medical Instrumentation) VP20-1994, Cardiovascular Implants-Vascular Prosthesis section 8.2.1.2, Method for Gravimetric Determination of Porosity. The pores in the polymer can have an average pore diameter from about 1 micron to about 100 microns. Preferably the average pore diameter is from about 1 micron to about 100 microns, and more preferably is from about 20 microns to about 70 microns. The average pore diameter is measured based on images from a scanning electron microscope (SEM). Formation of porous THORALON^{®} is described, for example, in U.S. Pat. No. 6,752,826 and 2003/0149471 A1. Non-porous THORALON^{®} can be formed by mixing the polyetherurethane urea (BPS-215) and the surface modifying additive (SMA-300) in a suitable solvent (described above) in the absence of the soluble particulate salt. The entire composition can be cast as a sheet, or coated onto an article such as a mandrel or a mold. In one example, the composition can be dried to remove the solvent.

Biocompatible polyurethane materials such as THORALON^{®} can be used in certain vascular applications and can be characterized by thromboresistance, high tensile strength, low water absorption, low critical surface tension, and good flex life. THORALON® is believed to be biostable and to be useful *in vivo* in long term blood contacting applications requiring biostability and leak resistance. Because of its flexibility, THOPALON^{®} is useful in larger vessels, such as the abdominal aorta, where elasticity and compliance is beneficial.

THORALON^{®} is described as an example of a biocompatible polyurethane, although other materials may also be used instead. A variety of other biocompatible polyurethanes ,may also be employed. These include polyurethane that preferably include a soft segment and include a hard segment formed from a diisocyanate and diamine. For example, polyurethane with soft segments such as PTMO, polyethylene oxide, polypropylene oxide, polycarbonate, polyolefin, polysiloxane (i.e. polydimethylsiloxane), and other polyether soft segments made from higher homologous series of diols may be used. Mixtures of any of the soft segments may also be used. The soft segments also may have either alcohol end groups or amine end groups. The molecular weight of the soft segments may vary from about 500 to about 5,000 g/mole.

The diisocyanate used as a component of the hard segment may be represented by the formula OCN-R-NCO, where -R- may be aliphatic, aromatic, cycloaliphatic or a mixture of aliphatic and aromatic moieties. Examples of diisocyanates include MDI, tetramethylene diisocyanate, hexamethylene diisocyanate, trimethyhexamethylene diisocyanate, tetramethylxylylene diisocyanate, 4,4'-dicyclohexylmethane diisocyanate, dimer acid diisocyanate, isophorone diisocyanate, metaxylene diisocyanate, diethylbenzene diisocyanate, decamethylene 1,10 diisocyanate, cyclohexylene 1,2-diisocyanate, 2,4-toluene diisocyanate, 2,6-toluene diisocyanate, xylene diisocyanate, m-phenylene diisocyanate, hexahydrotolylene diisocyanate (and isomers), naphthylene-1,5-diisocyanate, 1-methoxyphenyl 2,4-diisocyanate, 4,4'-biphenylene diisocyanate, 3,3'-dimethoxy-4,4'-biphenyl diisocyanate and mixtures thereof.

The diamine used as a component of the hard segment includes aliphatic amines, aromatic amines and amines contaning both aliphatic and aromatic moieties. For example, diamines include ethylene diamine, propane diamines, butanediamines, hexanediamines, pentane diamines, heptane diamines, octane diamines, m-xylylene diamine, 1,4-cyclohexane diamine, 2-methypentamethylene diamine, 4,4'-methylene dianiline, and mixtures thereof. The amines may also contain oxygen and/or halogen atoms in their structures.

Other applicable biocompatible polyurethanes include those using a polyol as a component of the hard segment. Polyols may be aliphatic, aromatic, cycloaliphatic or may contain a mixture of aliphatic and aromatic moieties. For example, the polyol may be ethylene glycol, diethylene glycol, triethylene glycol, 1,4-butanediol, 1,6-hexanediol, 1,8-octanediol, propylene glycols, 2,3-butylene glycol, dipropylene glycol, dibutylene glycol, glycerol, or mixtures thereof. Biocompatible polyurethanes modified with cationic, anionic and aliphatic side chains may also be used. See, for example, U.S. Pat. No. 5,017,664. Other biocompatible polyurethanes include: segmented polyurethanes, such as BIOSPAN^{®}; polycarbonate urethanes, such as BIONATE^{®}; and polyetherurethanes, such as ELASTHANE^{®}; (all available from POLYMER TECHNOLOGY GROUP, Berkeley, CA). Other biocompatible polyurethanes include polyurethanes having siloxane segments, also referred to as a siloxane-polyurethane. Examples of polyurethanes containing siloxane segments include polyether siloxane-polyurethanes, polycarbonate siloxane-polyurethanes, and siloxane-polyurethane ureas. Specifically, examples of siloxane-polyurethane include polymers such as ELAST-EON 2^{®} and ELAST-EON 3^{®} (AORTECH BIOMATERIALS, Victoria, Australia); polytetramethyleneoxide (PTMO) and polydimethylsiloxane (PPMS) polyether-based aromatic siloxane-polyurethanes such as PURSIL^{®}-10, -20, and -40 TSPU; PTMO and PDMS polyether-based aliphatic siloxane-polyurethanes such as PURSIL^{®} AL-5 and AL-10 TSPU; aliphatic, hydroxy-terminated polycarbonate and PDMS polycarbonate-based siloxane-polyurethanes such as CARBOSIL^{®}-10, -20, and -40 TSPU (all available from POLYMER TECHNOLOGY GROUP). The PURSIL^{®}, PURSIL^{®} -AL, and CARBOSIL^{®} polymers are thermoplastic elastomer urethane copolymers containing siloxane in the soft segment, and the percent siloxane in the copolymer is referred to in the grade name. For example, PURSIL^{®}-10 contains 10% siloxane. These polymers are synthesized through a multi-step bulk synthesis in which PDMS is incorporated into the polymer soft segment with PTMO (PURSIL^{®}) or an aliphatic hydroxy-terminated polycarbonate (CARBOSIL^{®}). The hard segment consists of the reaction product of an aromatic diisocyanate, MDI, with a low molecular weight glycol chain extender. In the case of PURSIL^{®}-AL the hard segment is synthesized from an aliphatic diisocyanate. The polymer chains are then terminated with a siloxane or other surface modifying end group. Siloxane-polyurethanes typically have a relatively low glass transition temperature, which provides for polymeric materials having increased flexibility relative to many conventional materials. In addition, the siloxane-polyurethane can exhibit high hydrolytic and oxidative stability, including improved resistance to environmental stress cracking. Examples of siloxane-polyurethanes are disclosed in U.S. Pat. Application Publication No. 2002/0187288 A1. In addition, any of these biocompatible polyurethanes may be end-capped with surface active end groups, such as, for example, polydimethylsiloxane, fluoropolymers, polyolefin, polyethylene oxide, or other suitable groups. See, for example the surface active end groups disclosed in U.S. Pat. No. 5,589,563.

In some embodiments of the present invention, it may be preferable to render at least a portion of a surface of the valve device antithrombogenic or thromboresistant. For example, a bioactive agent can be coated on the device surface of a valve leaflet or incorporated within a support frame or elliptical support. The bioactive agent can be a thromboresistant or antithrombogenic bioactive agent. A thromboresistant bioactive agent can be included in any suitable part of an implantable medical device. Selection of the type of thromboresistant bioactive, the portions of the medical device comprising the thromboresistant bioactive agent, and the manner of attaching the thromboresistant bioactive agent to the medical device can be chosen to perform a desired therapeutic function upon implantation. For example, a therapeutic bioactive agent can be combined with a biocompatible polyurethane, impregnated in an extracellular matrix material, incorporated in an implantable support frame or coated over any portion of the medical device. In one aspect, the implantable medical device can comprise one or more valve leaflets comprising a thromboresistant bioactive agent coated on the surface of the valve leaflet or impregnated in the valve leaflet. In another aspect, a thromboresistant bioactive material is combined with a biodegradable polymer to form a portion of an implantable frame.

Medical devices comprising an antithrombogenic bioactive agent are particularly preferred for implantation in areas of the body that contact blood. An antithrombogenic bioactive agent is any therapeutic agent that inhibits or prevents thrombus formation within a body vessel. The medical device can comprise any suitable antithrombogenic bioactive agent. Types of antithrombotic bioactive agents include anticoagulants, antiplatelets, and fibrinolytics. Anticoagulants are bioactive agents which act on any of the factors, cofactors, activated factors, or activated cofactors in the biochemical cascade and inhibit the synthesis of fibrin. Antiplatelet bioactive agents inhibit the adhesion, activation, and aggregation of platelets, which are key components of thrombi and play an important role in thrombosis. Fibrinolytic bioactive agents enhance the fibrinolytic cascade or otherwise aid is dissolution of a thrombus. Examples of antithrombotics include but are not limited to anticoagulants such as thrombin, Factor Xa, Factor VIIa and tissue factor inhibitors; antiplatelets such as glycoprotein IIb/IIIa, thromboxane A2, ADP-induced glycoprotein IIb/IIIa, and phosphodiesterase inhibitors; and fibrinolytics such as plasminogen activators, thrombin activatable fibrinolysis inhibitor (TAFI) inhibitors, and other enzymes which cleave fibrin. Further examples of antithrombotic bioactive agents include anticoagulants such as heparin, low molecular weight heparin, covalent heparin, synthetic heparin salts, coumadin, bivalirudin (hirulog), hirudin, argatroban, ximelagatran, dabigatran, dabigatran etexilate, D-phenalanyl-L-poly-L-arginyl, chloromethy ketone, dalteparin, enoxaparin, nadroparin, danaparoid, vapiprost, dextran, dipyridamole, omega-3 fatty acids, vitronectin receptor antagonists, DX-9065a, CI-1083, JTV-803, razaxaban, BAY 59-7939, and LY-51,7717; antiplatelets such as eftibatide, tirofiban, orbofiban, lotrafiban, abciximab, aspirin, ticlopidine, clopidogrel, cilostazol, dipyradimole, nitric oxide sources such as sodium nitroprussiate, nitroglycerin, S-nitroso and N-nitroso compounds; fibrinolytics such as alfimeprase, alteplase, anistreplase, reteplase, lanoteplase, monteplase, tenecteplase, urokinase, streptokinase, or phospholipid encapsulated microbubbles; and other bioactive agents such as endothelial progenitor cells or endothelial cells.

An antithrombogenic bioactive agent can be incorporated in or applied to portions of the implantable medical device by any suitable method that permits adequate retention of the bioactive agent material and the effectiveness thereof for an intended purpose upon implantation in the body vessel. The configuration of the bioactive agent on or in the medical device will depend in part on the desired rate of elution for the bioactive. Bioactive agents can be coated directly on the medical device surface or can be adhered to a medical device surface by means of a coating. For example, an antithrombotic bioactive agent can be blended with a polymer and spray or dip coated on the device surface. A bioactive agent material can be posited on the surface of the medical device and a porous coating layer can be posited over the bioactive agent material. The bioactive agent material can diffuse through the porous coating layer. Multiple porous coating layers and or pore size can be used to control the rate of diffusion of the bioactive agent material. The coating layer can also be nonporous wherein the rate of diffusion of the bioactive agent material through the coating layer is controlled by the rate of dissolution of the bioactive agent material in the coating layer. The bioactive agent material can also be dispersed throughout the coating layer, by for example, blending the bioactive agent with the polymer solution that forms the coating layer. If the coating layer is biostable, the bioactive agent can diffuse through the coating layer. If the coating layer is biodegradable, the bioactive agent is released upon erosion of the biodegradable coating layer. Bioactive agents may be bonded to the coating layer directly via a covalent bond or via a linker molecule which covalently links the bioactive agent and the coating layer. Alternatively, the bioactive agent may be bound to the coating layer by ionic interactions including cationic polymer coatings with anionic functionality on bioactive agent, or alternatively anionic polymer coatings with cationic functionality on the bioactive agent. Hydrophobic interactions may also be used to bind the bioactive agent to a hydrophobic portion of the coating layer. The bioactive agent may be modified to include a hydrophobic moiety such as a carbon based moiety, silicon-carbon based moiety or other such hydrophobic moiety. Alternatively, the hydrogen bonding interactions may be used to bind the bioactive agent to the coating layer.

Although the invention herein has been described in connection with a preferred embodiment thereof, it will be appreciated by those skilled in the art that additions, modifications, substitutions, and deletions not specifically described may be made without departing from the spirit and scope of the invention as defined in the appended claims. The scope of the invention is defined by the appended claims, and all devices that come within the meaning of the claims, either literally or by equivalence, are intended to be embraced therein.

## Claims

1. An elliptical prosthetic valve comprising an elliptical support structure (12) having a longitudinal axis (13), an interior surface and an outer surface, the interior surface defining an internal lumen containing the longitudinal axis, the internal lumen configured to conduct fluid flow therethrough, and the outer surface having an elliptical cross-sectional shape, the outer surface intersecting a first radial axis (12) at a first distance and intersecting a second radial axis (14) perpendicular to the first radial axis at a second distance that is less than the first distance, the first radial axis (12) and the second radial axis (14) being perpendicular to the longitudinal axis (13); wherein the elliptical support structure (20) comprises an elliptical ring comprising a first pair of collapse points (26) positioned at the points of intersection of the first radial axis with the elliptical ring; the elliptical ring being moveable from a planar configuration bisected by a first plane containing the first radial axis and the second radial axis, while moving the points of intersection of the elliptical ring with the second radial axis out of the first plane; and a flexible structure attached to the elliptical ring to regulate fluid flow through the prosthetic valve by moving in response to fluid flow within a body vessel.
the elliptical support structure **characterized by**:
(a) the first pair of collapse points (26) comprising a bioabsorbable material positioned at the points of intersection of the first radial axis with the elliptical ring, dissipation of the bioabsorbable material increasing the flexibility of the collapse points.

2. The elliptical prosthetic valve of claim 1, wherein the elliptical ring further comprises a second pair of collapse points (26) positioned at the points of intersection of the second radial axis (14) with the elliptical ring.

3. The elliptical prosthetic valve of at least one of claims 1 or 2, wherein the elliptical support structure (20) comprises a reinforcing frame portion (54) bridging the points of intersection of the elliptical ring with the first radial axis (12), the reinforcing frame portion being symmetrically bisected by a plane containing the longitudinal axis (13) and the first radial axis (12).

4. The elliptical prosthetic valve device of any preceding claim, wherein the elliptical prosthetic valve comprises a support frame (10) attached to the elliptical ring.

5. The elliptical prosthetic valve device of any preceding claim, comprising at least one imageable element (60) on said valve for orienting said valve within a body vessel.

6. The elliptical prosthetic valve of any preceding claim, , wherein the flexible structure operable to regulate fluid flow comprises a tubular flexible valve member defining a tubular lumen extending from an inlet end attached to the elliptical ring to a tapered end, the tubular lumen being contiguous with the internal lumen of the elliptical ring, the tubular lumen containing the longitudinal axis, and the tapered end defining a valve orifice having a cross sectional area that is less than the cross sectional area of the elliptical ring.

7. The elliptical prosthetic valve of any preceding claim, , wherein the flexible structure adapted to regulate fluid flow comprises a flexible valve leaflet attached to the elliptical ring, the flexible valve leaflet defining a valve orifice (38) contiguous with the internal lumen, the valve orifice (38) having an open configuration permitting fluid flow in a first direction along the longitudinal axis out of the internal lumen and a closed configuration substantially preventing fluid flow from entering the internal lumen along the longitudinal axis, the flexible valve leaflet (30) being moveable relative to the elliptical ring in response to the fluid flow within the internal lumen contacting the flexible valve leaflet.

8. The elliptical prosthetic valve device the claim 7, wherein said flexible valve leaflet (30) comprises a biocompatible polyurethane.

9. The elliptical prosthetic valve device of any preceding claim, , wherein the flexible structure comprises small intestine submucosa.

10. The elliptical prosthetic valve device at least one of any one of claims 7, 8 or 9, wherein the flexible structure includes one or more valve leaflets comprising small intestine submucosa.

11. The elliptical prosthetic valve device of any preceding claim, wherein the flexible structure is a pair of flexible valve leaflets (38) attached to the planar elliptical ring.

12. The elliptical prosthetic valve device of any preceding claim, wherein the flexible structure further comprises an antithrombogenic agent.

13. The elliptical prosthetic valve of any preceding claim, wherein the prosthetic valve further comprises a radially expandable frame (150) connected to the elliptical support structure.

14. The elliptical prosthetic valve of claim 13, wherein the frame (150) comprises a self-expanding material.

15. The prosthetic valve device of any preceding claim wherein the elliptical support ring comprises an attachment portion; said second radial axis (14) is shorter than said first radial axis (12) and said attachment portion is arranged for securing said valve in body vessel; and
wherein the flexible structure comprises at least one flexible valve leaflet (30) attached to said elliptical support structure (20), the valve leaflet (38) comprising a material selected from the group consisting of: a biocompatible polyurethane and an extracellular matrix material.

16. The elliptical prosthetic valve of claim 15, wherein the attachment portion comprises an extracellular matrix material.

17. The elliptical prosthetic valve of any preceding claim, where the elliptical prosthetic valve is designed for introduction to a vein, and the flexible structure (20) comprises a flexible valve leaflet (30) attached to the elliptical support structure, the flexible valve leaflet (38) defining a valve orifice (34) contiguous with the internal lumen, the valve orifice having an open configuration permitting fluid flow in a first direction along the longitudinal axis out of the internal lumen and a closed configuration substantially preventing fluid flow from entering the internal lumen along the longitudinal axis, the flexible valve leaflet (38) being moveable relative to the elliptical ring in response to the fluid flow within the internal lumen contacting the flexible valve leaflet.

18. The elliptical prosthetic valve of claim 17, wherein the planar elliptical ring is moveable from a planar configuration to a bent configuration by bending the planar elliptical ring at the first pair of collapse points while moving the points of intersection of the planar elliptical ring with the second radial axis out of the plane containing the first radial axis (12) and the second radial axis (14).

19. The elliptical prosthetic valve of claim 18 or 19, wherein the flexible structure is arranged to regulate fluid flow through the interior lumen includes two or more flexible valve leaflets (38) comprising an extracellular matrix material.

20. The elliptical prosthetic valve of at least one of claims 1, 7, 8, 10, 11, 15, 17 or 20, where the flexible valve leaflet (38) comprises a remodelable material.

## Patentansprüche

1. Elliptische künstliche Klappe, die eine elliptische Stützstruktur (20) umfasst, die eine Längsachse (13), eine Innenfläche und eine Außenfläche hat, wobei die Innenfläche ein inneres Lumen definiert, das die Längsachse enthält, wobei das innere Lumen dafür konfiguriert ist, ein Fluid durch dasselbe zu leiten, und die Außenfläche eine elliptische Querschnittsform hat, wobei die Außenfläche eine erste radiale Achse (12) in einem ersten Abstand schneidet und eine zweite radiale Achse (14), senkrecht zu der ersten radialen Achse, in einem zweiten Abstand, der geringer ist als der erste Abstand, schneidet, wobei die erste radiale Achse (12) und die zweite radiale Achse (14) senkrecht zu der Längsachse (13) sind, wobei die elliptische Stützstruktur (20) einen elliptischen Ring, der ein erstes Paar von Faltpunkten (26) umfasst, die an den Schnittpunkten der ersten radialen Achse mit dem elliptischen Ring angeordnet sind, wobei der elliptische Ring bewegt werden kann aus einer ebenen Konfiguration, die halbiert wird durch eine erste Ebene, welche die erste radiale Achse und die zweite radiale Achse enthält, während die Schnittpunkte des elliptischen Rings mit der zweiten radialen Achse aus der ersten Ebene bewegt werden, und eine flexible Struktur, die an dem elliptischen Ring befestigt ist, um durch ein Bewegen als Reaktion auf einen Fluidstrom innerhalb eines Körpergefäßes den Fluidstrom durch die künstliche Klappe zu regulieren, umfasst,
wobei die elliptische Stützstruktur **dadurch gekennzeichnet ist,**
(a) **dass** das erste Paar von Faltpunkten (26), die ein biologisch absorbierbares Material umfassen, an den Schnittpunkten der ersten radialen Achse mit dem elliptischen Ring angeordnet sind, wobei die Auflösung des biologisch absorbierbaren Materials die Flexibilität der Faltpunkte steigert.

2. Elliptische künstliche Klappe nach Anspruch 1, wobei der elliptischen Ring ferner ein zweites Paar von Faltpunkten (26) umfasst, die an den Schnittpunkten der zweiten radialen Achse (14) mit dem elliptischen Ring angeordnet sind.

3. Elliptische künstliche Klappe nach wenigstens einem der Ansprüche 1 oder 2, wobei die elliptische Stützstruktur (20) einen verstärkenden Rahmenabschnitt (54) umfasst, der die Schnittpunkte des elliptischen Rings mit der ersten radialen Achse (12) überbrückt, wobei der verstärkende Rahmenabschnitt symmetrisch durch eine Ebene halbiert wird, welche die Längsachse (13) und die erste radiale Achse (12) enthält.

4. Elliptische künstliche Klappe nach einem der vorhergehenden Ansprüche, wobei die elliptische künstliche Klappe einen Stützrahmen (10) umfasst, der an dem elliptischen Ring befestigt ist.

5. Elliptische künstliche Klappe nach einem der vorhergehenden Ansprüche, die wenigstens ein abbildbares Element (60) an der Klappe zum Ausrichten der Klappe innerhalb eines Körpergefäßes umfasst.

6. Elliptische künstliche Klappe nach einem der vorhergehenden Ansprüche, wobei die flexible Struktur, die funktionsfähig ist, um einen Fluidstrom zu regulieren, ein röhrenförmiges flexibles Klappenelement umfasst, das ein röhrenförmiges Lumen definiert, das sich von einem an dem elliptischen Ring befestigten Einlassende bis zu einem verjüngten Ende erstreckt, wobei das röhrenförmige Lumen mit dem inneren Lumen des elliptischen Rings zusammenhängt, wobei das röhrenförmige Lumen die Längsachse enthält und das verjüngte Ende eine Klappenöffnung definiert, die eine Querschnittsfläche hat, die geringer ist als die Querschnittsfläche des elliptischen Rings.

7. Elliptische künstliche Klappe nach einem der vorhergehenden Ansprüche, wobei die flexible Struktur, die dafür eingerichtet ist, einen Fluidstrom zu regulieren, ein an dem elliptischen Ring befestigtes flexibles Klappensegel umfasst, wobei das flexible Klappensegel eine mit dem inneren Lumen zusammenhängende Klappenöffnung (38) definiert, wobei die Klappenöffnung (38) eine offene Konfiguration, die einen Fluidstrom in einer ersten Richtung entlang der Längsachse aus dem inneren Lumen ermöglicht, und eine geschlossene Konfiguration, die im Wesentlichen verhindert, dass ein Fluidstrom entlang der Längsachse in das innere Lumen eintritt, aufweist, wobei das flexible Klappensegel (30) als Reaktion auf den Fluidstrom innerhalb des inneren Lumens, der das flexible Klappensegel berührt, im Verhältnis zu dem elliptischen Ring bewegt werden kann.

8. Elliptische künstliche Klappe nach Anspruch 7, wobei das flexible Klappensegel (30) ein biologisch verträgliches Polyurethan umfasst.

9. Elliptische künstliche Klappe nach einem der vorhergehenden Ansprüche, wobei die flexible Struktur Dünndarm-Schleimhaut umfasst.

10. Elliptische künstliche Klappe nach mindestens einem der Ansprüche 7, 8 oder 9, wobei die flexible Struktur ein oder mehrere Klappensegel umfasst, die Dünndarm-Schleimhaut umfassen.

11. Elliptische künstliche Klappe nach einem der vorhergehenden Ansprüche, wobei die flexible Struktur ein Paar von flexiblen Klappensegeln (30), die an dem ebenen elliptischen Ring befestigt sind.

12. Elliptische künstliche Klappe nach einem der vorhergehenden Ansprüche, wobei die flexible Struktur ferner ein antithrombotisches Mittel umfasst.

13. Elliptische künstliche Klappe nach einem der vorhergehenden Ansprüche, wobei die künstliche Klappe ferner einen in Radialrichtung ausdehnbaren Rahmen (150) umfasst, der mit der elliptischen Stützstruktur verbunden ist.

14. Elliptische künstliche Klappe nach Anspruch 13, wobei der Rahmen (150) ein selbstausdehnendes Material umfasst.

15. Künstliche Klappe nach einem der vorhergehenden Ansprüche, wobei der elliptische Stützring einen Befestigungsabschnitt umfasst, wobei die zweite radiale Achse (14) kürzer ist als die erste radiale Achse (12) und der Befestigungsabschnitt zum Befestigen der Klappe in einem Körpergefäß angeordnet ist und
wobei die flexible Struktur wenigstens ein flexibles Klappensegel (30) umfasst, das an der elliptischen Stützstruktur (20) befestigt ist, wobei das flexible Klappensegel (30) ein Material umfasst, das ausgewählt ist aus der Gruppe, die aus einem biologisch verträglichen Polyurethan und einem extrazellulären Matrixmaterial besteht.

16. Elliptische künstliche Klappe nach Anspruch 15, wobei der Befestigungsabschnitt ein extrazelluläres Matrixmaterial umfasst.

17. Elliptische künstliche Klappe nach einem der vorhergehenden Ansprüche, wobei die elliptische künstliche Klappe für die Einführung in eine Vene gestaltet ist und die flexible Struktur (20) ein flexibles Klappensegel (30) umfasst, das an der elliptischen Stützstruktur befestigt ist, wobei das flexible Klappensegel (30) eine mit dem inneren Lumen zusammenhängende Klappenöffnung (38) definiert, wobei die Klappenöffnung eine offene Konfiguration, die einen Fluidstrom in einer ersten Richtung entlang der Längsachse aus dem inneren Lumen ermöglicht, und eine geschlossene Konfiguration, die im Wesentlichen verhindert, dass ein Fluidstrom entlang der Längsachse in das innere Lumen eintritt, aufweist, wobei das flexible Klappensegel (30) als Reaktion auf den Fluidstrom innerhalb des inneren Lumens, der das flexible Klappensegel berührt, im Verhältnis zu dem elliptischen Ring bewegt werden kann.

18. Elliptische künstliche Klappe nach Anspruch 17, wobei der ebene elliptische Ring von einer ebenen Konfiguration zu einer gebogenen Konfiguration bewegt werden kann durch das Biegen des ebenen elliptischen Rings an dem ersten Paar von Faltpunkten, während die Schnittpunkte des ebenen elliptischen Rings mit der zweiten radialen Achse aus der Ebene bewegt werden, welche die erste radiale Achse (12) und die zweite radiale Achse (14) enthält.

19. Elliptische künstliche Klappe nach Anspruch 18 oder 19, wobei die flexible Struktur, die dafür angeordnet ist, einen Fluidstrom durch das innere Lumen zu regulieren, zwei oder mehr flexible Klappensegel (30) einschließt, die ein extrazelluläres Matrixmaterial umfassen.

20. Elliptische künstliche Klappe nach wenigstens einem der Ansprüche 1, 7, 8, 10, 11, 15, 17 oder 20, wobei das flexible Klappensegel (30) ein umformbares Material umfasst.

## Revendications

1. Valvule prothétique elliptique comportant une structure (20) de support elliptique présentant un axe (13) longitudinal, une surface intérieure et une surface externe, la surface intérieure délimitant une lumière interne contenant l'axe longitudinal, la lumière interne étant configurée pour y conduire l'écoulement de fluide, et la surface externe présentant une forme en coupe elliptique, la surface externe croisant un premier axe (12) radial à une première distance et croisant un second axe (14) radial perpendiculaire au premier axe radial à une seconde distance qui est inférieure à la première distance, le premier axe (12) radial et le second axe (14) radial étant perpendiculaires à l'axe (13) longitudinal ; dans laquelle la structure (20) de support elliptique comporte un anneau elliptique comportant une première paire de points (26) d'affaissement positionnés aux points d'intersection du premier axe radial avec l'anneau elliptique ; l'anneau elliptique étant mobile depuis une configuration plane coupée en deux par un premier plan contenant le premier axe radial et le second axe radial, tout en déplaçant les points d'intersection de l'anneau elliptique avec le second axe radial hors du premier plan ; et une structure souple fixée à l'anneau elliptique pour réguler l'écoulement de fluide dans la valvule prothétique en se déplaçant en réponse à l'écoulement de fluide à l'intérieur d'un vaisseau corporel,
la structure de support elliptique étant **caractérisée par** :
(a) la première paire de points (26) d'affaissement comportant une matière bioabsorbable positionnée aux points d'intersection du premier axe radial avec l'anneau elliptique, la dissolution de la matière bioabsorbable augmentant la flexibilité des points d'affaissement.

2. Valvule prothétique elliptique selon la revendication 1, dans laquelle l'anneau elliptique comporte en outre une seconde paire de points (26) d'affaissement positionnés aux points d'intersection du second axe (14) radial avec l'anneau elliptique.

3. Valvule prothétique elliptique selon au moins l'une des revendications 1 et 2, dans laquelle la structure (20) de support elliptique comporte une partie (54) formant cadre de renfort comblant l'espace entre les points d'intersection de l'anneau elliptique avec le premier axe (12) radial, la partie formant cadre de renfort étant coupée en deux symétriquement par un plan contenant l'axe (13) longitudinal et le premier axe (12) radial.

4. Dispositif de valvule prothétique elliptique selon l'une quelconque des revendications précédentes, dans lequel la valvule prothétique elliptique comporte un cadre (10) de support fixé à l'anneau elliptique.

5. Dispositif de valvule prothétique elliptique selon l'une quelconque des revendications précédentes, comportant au moins un élément (60) visualisable par imagerie sur ladite valvule pour orienter ladite valvule à l'intérieur d'un vaisseau corporel.

6. Valvule prothétique elliptique selon l'une quelconque des revendications précédentes, dans laquelle la structure souple servant à réguler l'écoulement de fluide comporte un élément de valvule souple tubulaire délimitant une lumière tubulaire qui s'étend d'une extrémité d'orifice d'admission fixée à l'anneau elliptique à une extrémité conique, la lumière tubulaire étant contiguë à la lumière interne de l'anneau elliptique, la lumière tubulaire contenant l'axe longitudinal, et l'extrémité conique délimitant un orifice valvulaire présentant une superficie de section qui est inférieure à la superficie de section de l'anneau elliptique.

7. Valvule prothétique elliptique selon l'une quelconque des revendications précédentes, dans laquelle la structure souple conçue pour réguler l'écoulement de fluide comporte un feuillet valvulaire souple fixé à l'anneau elliptique, le feuillet valvulaire souple délimitant un orifice (38) valvulaire contigu à la lumière interne, l'orifice (38) valvulaire présentant une configuration ouverte permettant l'écoulement de fluide dans une première direction le long de l'axe longitudinal hors de la lumière interne et une configuration fermée empêchant sensiblement l'écoulement de fluide d'entrer dans la lumière interne le long de l'axe longitudinal, le feuillet (30) valvulaire souple étant mobile par rapport à l'anneau elliptique en réponse à l'écoulement de fluide à l'intérieur de la lumière interne venant en contact avec le feuillet valvulaire souple.

8. Dispositif de valvule prothétique elliptique selon la revendication 7, dans lequel ledit feuillet (30) valvulaire souple comporte un polyuréthane biocompatible.

9. Dispositif de valvule prothétique elliptique selon l'une quelconque des revendications précédentes, dans lequel la structure souple comporte une sousmuqueuse d'intestin grêle.

10. Dispositif de valvule prothétique elliptique selon au moins l'une quelconque des revendications 7, 8 et 9, dans lequel la structure souple comprend un ou plusieurs feuillets valvulaires comportant une sous-muqueuse d'intestin grêle.

11. Dispositif de valvule prothétique elliptique selon l'une quelconque des revendications précédentes, dans lequel la structure souple est une paire de feuillets (30) valvulaires souples fixés à l'anneau elliptique plan.

12. Dispositif de valvule prothétique elliptique selon l'une quelconque des revendications précédentes, dans lequel la structure souple comporte en outre un agent antithrombogène.

13. Valvule prothétique elliptique selon l'une quelconque des revendications précédentes, dans laquelle la valvule prothétique comporte en outre un cadre (150) radialement expansible relié à la structure de support elliptique.

14. Valvule prothétique elliptique selon la revendication 13, dans laquelle le cadre (150) comporte une matière auto-expansible.

15. Dispositif de valvule prothétique selon l'une quelconque des revendications précédentes dans lequel l'anneau de support elliptique comporte une partie de fixation ; ledit second axe (14) radial est plus court que ledit premier axe (12) radial et ladite partie de fixation est agencée de manière à immobiliser ladite valvule dans un vaisseau corporel ; et
dans lequel la structure souple comporte au moins un feuillet (30) valvulaire souple fixé à ladite structure (20) de support elliptique, le feuillet (30) valvulaire comportant une matière choisie dans le groupe constitué des éléments suivants : un polyuréthane biocompatible et une matière de matrice extracellulaire.

16. Valvule prothétique elliptique selon la revendication 15, dans laquelle la partie de fixation comporte une matière de matrice extracellulaire.

17. Valvule prothétique elliptique selon l'une quelconque des revendications précédentes, la valvule prothétique elliptique étant conçue pour être introduite dans une veine, et la structure (20) souple comporte un feuillet (30) valvulaire souple fixé à la structure de support elliptique, le feuillet (30) valvulaire souple définissant un orifice (38) valvulaire contigu à la lumière interne, l'orifice valvulaire présentant une configuration ouverte permettant l'écoulement de fluide dans une première direction le long de l'axe longitudinal hors de la lumière interne et une configuration fermée empêchant sensiblement l'écoulement de fluide d'entrer dans la lumière interne le long de l'axe longitudinal, le feuillet (30) valvulaire souple étant mobile par rapport à l'anneau elliptique en réponse à l'écoulement de fluide à l'intérieur de la lumière interne venant en contact avec le feuillet valvulaire souple.

18. Valvule prothétique elliptique selon la revendication 17, dans laquelle l'anneau elliptique plan est mobile d'une configuration plane à une configuration pliée en pliant l'anneau elliptique plan au niveau de la première paire de points d'affaissement tout en déplaçant les points d'intersection de l'anneau elliptique plan avec le second axe radial hors du plan contenant le premier axe (12) radial et le second axe (14) radial.

19. Valvule prothétique elliptique selon la revendication 18 ou 19, dans laquelle la structure souple qui est disposée de façon à réguler l'écoulement de fluide dans la lumière intérieure comprend deux feuillets (30) valvulaires souples ou plus et comporte une matière de matrice extracellulaire.

20. Valvule prothétique elliptique selon au moins l'une des revendications 1, 7, 8, 10, 11, 15, 17 et 20, le feuillet (30) valvulaire souple comportant une matière refaçonnable.
